(19) Europäisches Patentamt
European Patent Office
Office européen des brevets

(11) Publication number: 0 108 627
A2

# (12) EUROPEAN PATENT APPLICATION

(21) Application number: 83306742.4

(22) Date of filing: 04.11.83

(51) Int. Cl.³: C 07 D 501/20
A 61 K 31/545

(30) Priority: 05.11.82 JP 195036/82

(43) Date of publication of application:
16.05.84 Bulletin 84/20

(84) Designated Contracting States:
AT BE CH DE FR GB IT LI NL SE

(71) Applicant: SUMITOMO CHEMICAL COMPANY, LIMITED
15 Kitahama 5-chome Higashi-ku
Osaka-shi Osaka-fu(JP)

(72) Inventor: Masai, Naruhito
9-9, Niwashirodai-4-cho
Sakai-shi(JP)

(72) Inventor: Ueda, Shinji
2-401, Ryodocho-4-chome
Nishinomiya-shi(JP)

(72) Inventor: Sano, Akihiko
7-22, Uenonishi-1-chome
Toyonaka-shi(JP)

(72) Inventor: Yamada, Hirotada
1-1-808, Takahatacho
Nishinomiya-shi(JP)

(72) Inventor: Suzaki, Hidekuni
10-3-351, Sonehigashinocho-2-chome
Toyonaka-shi(JP)

(72) Inventor: Okuda, Takao
10-3-337, Sonehigashinocho-2-chome
Toyonaka-shi(JP)

(72) Inventor: Fukumura, Masataka
7-15, Hikarigaoka-2-chome
Takarazuka-shi(JP)

(74) Representative: Goldin, Douglas Michael et al,
J.A. KEMP & CO. 14, South Square Gray's Inn
London WC1R 5EU(GB)

## (54) Cephalosporin derivatives and process for producing the same.

(57) A compound of the general formula:

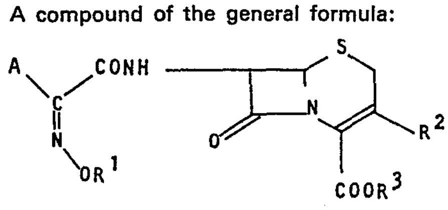

wherein A represents an unsubstituted thiazolyl or substituted thiazolyl residue having at least one substituent which is halogen, alkyl, amino or acylamido; $R^1$ represents hydrogen or alkyl, carboxymethyl or alkoxycarbonylmethyl, $R^2$ represents hydrogen or methyl, hydroxymethyl, acyloxymethyl or alkylthiomethyl, or represents thiadiazolylthiomethyl optionally alkyl substituted, or represents tetrazolylthiomethyl residue optionally substituted by alkyl, alkylaminoalkyl or carboxymethyl; and $R^3$ represents unsubstituted allyl or substituted allyl having at least one substituent which is halogen, alkyl, cycloalkyl, haloalkyl, acyloxy alkyl, alkoxycarbonyl, phenyl or substituted phenyl, or salts thereof have excellent chemical stability and are very satisfactorily absorbed to exhibit an excellent therapeutic effect on the patients having bacterial infectious diseases when administered by a method other than injection, such as oral administration. Various methods are disclosed for the preparation and formulation of the new compounds.

EP 0 108 627 A2

# CEPHALOSPORIN DERIVATIVES AND PROCESS FOR PRODUCING THE SAME

The present invnetion relates to novel cephem compounds. More particularly, the invention relates to compounds represented by the following general formula (I) and their salts:

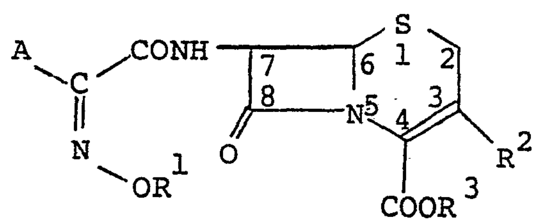

[I]

wherein A represents unsubstituted thiazolyl residue or substituted thiazolyl residue having at least one substituent selected from the group consisting of halogen atom, lower alkyl residue, amino residue and acylamido residue; $R^1$ represents hydrogen atom, lower alkyl residue, carboxymethyl residue or lower alkoxycarbonylmethyl residue; $R^2$ represents hydrogen atom, methyl residue, hydroxymethyl residue, acyloxymethyl residue or lower alkylthiomethyl residue or represents thiadiazolylthiomethyl residue optionally substituted by lower alkyl residue or tetrazolylthiomethyl residue optionally substituted by lower alkyl residue, lower alkylaminoalkyl residue or carboxymethyl residue; and $R^3$ represents unsubstituted allyl residue or substitute allyl residue having at least one substituent selected from the group consisting of halogen atom, lower alkyl residue, lower cycloalkyl residue, lower haloalkyl residue, acyloxy-(lower alkyl) residue, lower alkoxycarbonyl residue, phenyl residue and substituted phenyl residue.

When the thiazolyl residue A of the compounds of the invention is in an equilibrium with thiazolinyl residue as expressed by the following scheme:

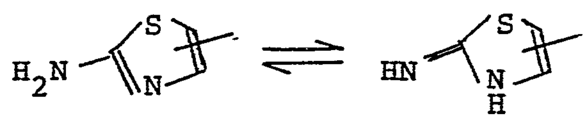

these tautomers are usually regarded as identical substances. In the invention, therefore, both the tautomers are inclusively referred to as "thiazolyl residue" for convenience and expressed by, for example: 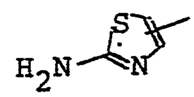 However, it should be noted that this formula expresses the above-mentioned tautomers inclusively and that they are both included in the invention.

The so-called second and third generations type cephalosporin compounds recently developed are not readily absorbable at the gastrointestinal tract in spite of their marked efficacy for the treatment of patients having bacterial infections, so that they are administered by injection today. Thus, development of a cephalosporin compound capable of displaying a sufficient therapeutic effect even if administered by a method other than injection, such as oral administration, and having a widely adaptable and strong antibacterial activity is intensely desired by patients having various bacterial infections diseases.

As prior arts regarding oral cephalosproin, we

can refer to, for example, Japanese Patent Kokai (Laid-Open) No. 160,782/80, No. 8,391/81, No. 158,788/81 and No. 154,190/82. However, none of these arts is yet practically employed.

The present inventors have long conducted an energetic research for a cephalosporin compound which is quite excellent in chemical stability and satisfactorily absorbed by administration methods other than injection, such as oral administration, to display an excellent therapeutic effect. As the result, the inventors have discovered a fact that the above-mentioned cephalosporin compounds represented by general formula [I] or their salts are very stable chemically and can bring about a sufficient therapeutic effect without marked side effect even when administered by an administration method other than injection, such as oral administration. Based on this discovery, the present invention has been accomplished.

The compounds of the invention further have the following characteristic features. Thus, since the compounds of the invention scarcely exhibit an antibacterial activity in themselves, they hardly affect the intestinal bacteria when administered orally, and only the absorbed portion is rapidly hydrolyzed in the living body to exhibit an antibacterial action. Further, when given orally, they are rapidly absorbed and the ester residue existing at the 4-position of the cephalosporin skeleton is hydrolyzed to yield the corresponding carboxylic acid, which appears in the blood and thereafter is excreted in the urine.

In the description of this specification, various residues included in the scope of the invention have the following significances.

"Halogen" means chlorine, bromine, iodine or fluorine.

"Lower alkyl" means lower alkyls having 1-6 carbon atoms such as methyl, ethyl, n-propyl, isopropyl, butyl, isobutyl, tertiary butyl, pentyl, hexyl and the like, and preferably means lower alkyls having 1-4 carbon atoms such as methyl, ethyl, n-propyl, isopropyl, butyl, isobutyl, tertiary butyl and the like, and more preferably means lower alkyls having 1-3 carbon atoms such as methyl, ethyl, n-propyl, isopropyl.

"Lower cycloalkyl" means lower cycloalkyls having 3-6 carbon atoms such as cyclopropyl, cyclobutyl, cyclopentyl, cyclohexyl and the like.

"Acylamido" means amino of which hydrogen has been substituted by acyl residue defined later, and preferably means formamido, acetylamido, propionylamido and the like.

"Acyl" means alkanoyl having 1-7 carbon atoms such as formyl, acetyl, propionyl, butyryl, isobutyryl, valeryl, isovaleryl, oxalo, succinyl, pivaloyl, hexanoyl and the like; alkoxycarbonyl having 1-7 carbon atoms such as methoxycarbonyl, ethoxycarbonyl, propoxycarbonyl, isopropoxycarbonyl, butoxycarbonyl, tertiary butoxycarbonyl, pentyloxycarbonyl, tertiary pentyloxycarbonyl, hexyloxycarbonyl and the like; aralkoxycarbonyl such as

benzyloxycarbonyl, phenethyloxycarbonyl and the like; aroyl such as benzoyl tolyol and the like; alkanesulfornyl having 1-6 carbon atoms such as metyl, ethanesulfonyl, 1-methylethanesulfonyl, propanesulfonyl, butanesulfonyl and the like; arenesulfonyl such as benzenesulfonyl, tosyl, naphthalenesulfonyl and the like. Preferably, it means formyl, acetyl, butyryl, isobutyryl, benzoyl, mesyl and the like.

The "lower alkyl part" in the lower alkoxycarbonylmethyl has the same meaning as the above-mentioned lower alkyl. Preferable examples of the lower alkoxycarbonylmethyl include methoxycarbonylmethyl, ethoxycarbonylmethyl, n-propoxycarbonylmethyl, isopropoxycarbonylmethyl, tertiary butoxycarbonylmethyl and the like.

"Acyloxymethyl residue" means alkanoyloxymethyl having 1-9 carbon atoms such as formyloxymethyl, acetyloxymethyl, propionyloxymethyl, butyryloxymethyl, isobutyryloxymethyl, valeryloxymethyl, isovaleryloxymethyl, pivaloyloxymethyl, hexanoyloxymethyl, heptanoyloxymethyl, octanoyloxymethyl and the like; aroyloxymethyl such as benzoyloxymethyl, toluoyloxymethyl and the like; alkanesulfonyloxymethyl having 1-7 carbon atoms such as methanesulfonyloxymethyl, ethanesulfonyloxymethyl, propanesulfonyloxymethyl and the like; arenesulfonyloxymethyl such as benzenesulfonyloxymethyl, tosyloxymethyl and the like, and the like. Preferably, it means formyloxymethyl, acetyloxymethyl, propionyloxymethyl and the like.

The "lower alkyl part" in the lower alkylthiomethyl has the same meaning as the above-mentioned lower alkyl. Preferable examples of the lower alkylthiomethyl include methylthiomethyl, ethylthiomethyl, n-propylthiomethyl, isoprophylthiomethyl and the like.

Preferable examples of the "thiadiazolylthiomethyl optionally substituted by lower alkyl" and "tetrazolylthiomethyl optionally substituted by lower alkyl" include 1,3,4,-thiadiazolylthiomethyl, 5-methyl-1,3,4-thiadiazolylthiomethyl, 1-methyl-1,2,3,4-tetrazolylthiomethyl, 1-ethyl-1,2,3,4-tetrazolylthiomethyl and the like. In the "tetrazolylthiomethyl residue optionally substituted by (lower alkyl)-aminoalkyl" the (lower alkyl)-aminoalkyl means the lower alkyl defined above having an amino residue substitued by the lower alkyl defined above. Its preferable examples include 1-[2-(dimethylamino)-ethyl]-1,2,3,4-tetrazolylthiomethyl and the like.

"Lower haloalkyl" means the lower alkyl defined above having the halogen defined above. Its preferable examples include methyl, ethyl, n-propyl, isopropyl and the like substituted by the halogen defined above. Its more preferable examples include methyl, ethyl, n-propyl, isopropyl and the like having one or more chlorine or fluorine atom(s).

The "acyl" and "lower alkyl" in the acyloxy-(lower alkyl) have the same meanings as mentioned above. Preferable examples of said acyloxy(lower alkyl) include formyloxymethyl, formyloxyethyl, formyloxypropyl,

acetyloxymethyl, acetyloxyethyl, acetyloxypropyl, propionyloxymethyl, propionyloxyethyl, propionyloxypropyl and the like.

The "lower alkyl" in the lower alkoxycarbonyl has the same meaning as defined in the paragraph of lower alkyl. Examples of said lower alkoxycarbonyl include methoxycarbonyl, ethoxycarbonyl, propoxycarbonyl, isopropoxycarbonyl, butoxycarbonyl, tertiary butoxycarbonyl, pentyloxycarbonyl, tertiary pentyloxycarbonyl, hexyloxycarbonyl and the like, and its preferable examples include methoxycarbonyl, ethoxycarbonyl, propoxycarbonyl and the like.

"Substituted phenyl" means a phenyl having a substituent selected from the group consisting of halogen, hydroxy, lower alkyl, lower alkoxy, lower haloalkylmercapto, lower alkylthio, amino, lower alkylamino, carboxy, lower alkylcarbonyl, acyl, nitro, nitrilo, carbamyl and combinations thereof. Here, the lower alkyl part in the lower alkoxy has the same meaning as defined in the paragraph of lower alkyl, and preferable examples of said lower alkoxy include methoxy, ethoxy, n-propoxy, isopropoxy and the like.

In the substituted phenyl, preferable examples of the substituent include halogen, lower alkyl, hydroxy, amino, lower alkoxy and the like.

Preferable examples of the compound of the invention include those represented by general formula [I] wherein:

A is unsubstituted or amino-substituted thiazolyl residue;

$R^1$ is hydrogen atom, lower alkyl residue or carboxymethyl residue;

$R^2$ is thiadiazolylthiomethyl residue optionally substituted by hydrogen atom, methyl residue or lower alkyl residue or tetrazolylthiomethyl residue optionally substituted by lower alkyl residue; and

$R^3$ is allyl residue having a substituent selected from the group consisting of halogen atom, lower alkyl residue, phenyl residue and substituted phenyl residue.

More preferable examples of the compound of the invention are those in which:

A is a residue represented by 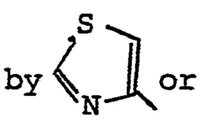 or 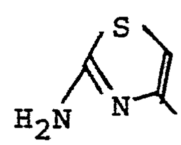

$R^1$ is hydrogen atom, methyl residue or carboxymethyl residue;

$R^2$ is hydrogen atom, methyl residue or a residue represented by 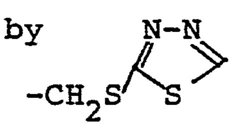 , 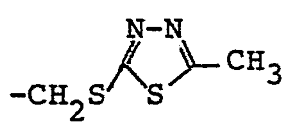 or

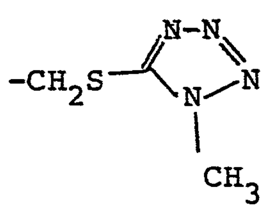 ; and

$R^3$ is allyl residue having a substituent selected from the group consisting of chlorine atom, methyl residue, ethyl residue, propyl residue, phenyl residue and substituted phenyl residue substituted by lower alkyl residue, lower alkoxy residue or halogen atom.

In general formula [I], the term "salt" means a salt of the amino residue, if the amino group exists in A, or a salt of amino or carboxyl residue existing in other position. As the salt of amino residue, any salts may be used so far as the salts can be formed from the amino residue. Examples of the salts include salts of mineral acid such as HX salt (X is halogen atom), sulfate, phosphate, nitrate and the like, and salts of organic acid such as acetic acid, propionic acid, citric acid, malic acid, tartaric acid, succinic acid, ascorbic acid, mandelic acid, methanesulfonic acid, ethanesulfonic acid, p-toluenesulfonic acid and the like. As the salt of carboxylic acid, any salts may be used so far as the salts can be formed from the carboxyl residue. Examples of the salts include metallic salts such as lithium salt, sodium salt, potassium salt, calcium salt, magnesium salt and the like; as well as triethylamine salt, dicyclohexylamine salt, dimethylbenzylamine salt and the like.

The compounds of the invention represented by the general formula [I] can be produced by either of the processes shown below.

Process (a): Acylation

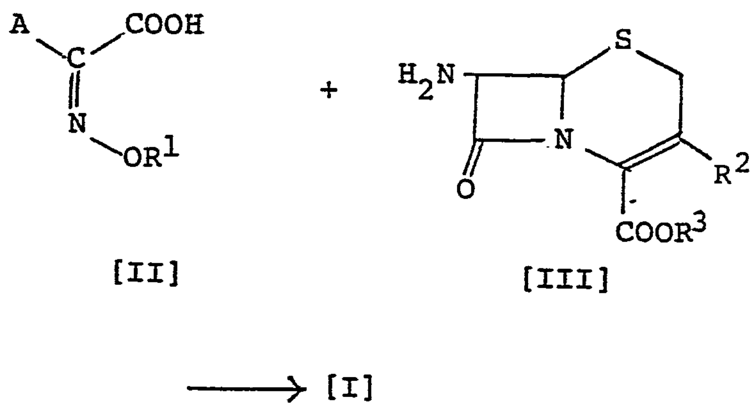

wherein A, $R^1$, $R^2$ and $R^3$ are as defined above.

The compound of the invention can be produced by reacting a compound represented by general formula [II] or its reactive derivative at carboxyl group or its salt with a compound represented by general formula [III] or its reactive derivative at the 7-amino group or its salt.

As the reactive derivative of the compound of general formula [II] or its salt, reactive derivatives conventionally used in such acylation reactions, such as acid halide, acid anhydride, active amide, active ester and the like can be referred to. More particularly, there can be referred to acid halides such as acid chloride, acid bromide and the like; mixed anhydrides of substituted phosphoric acids such as mixed anhydride of dialkyl phosphate, mixed anhydride of phenyl phosphate, mixed anhydride of diphenyl phosphate, mixed anhydride of dibenzyl phosphate, mixed anhydride of halogeno-phosphoric acid and the like; acid anhydrides such as mixed anhydride of dialkyl phosphite, mixed anhydride of sulfurous acid,

mixed anhydride of thiosulfuric acid, mixed anhydride of sulfuric acid, mixed anhydride of alkyl carbonate, mixed anhydrides of fatty acids (e.g. pivalic acid, pentanoic acid, isopentanoic acid, 2-ethylbutanoic acid, trichloroacetic acid), mixed anhydrides of aromatic carboxylic acids (e.g. benzoic acid), symmetric acid anhydrides and the like; acid amides with imidazole, 4-substituted imidazole, dimethylpyrazole, triazole, tetrazole and the like; esters such as cyanomethyl ester, methoxymethyl ester, vinyl ester, p-nitrophenyl ester, 2,4-dinitrophenyl ester, trichlorophenyl ester, pentachlorophenyl ester, mesylphenyl ester, phenyl-azophenyl ester, phenylthio ester, p-nitrophenylthio ester, p-cresylthio ester, carboxymethylthio ester, pyranyl ester, pyridyl ester, piperidyl ester, 8-quinolylthio ester and the like; and esters with N,N-dimethylhydroxylamine, N-hydroxysuccinimide, N-hydroxyphthalimide, 1-hydroxybenzotriazole, 1-hydroxy-6-chloro-1H-benzotriazole, 8-hydroxyquinoline and the like.

The reactive derivative, at the position of 7-amino group, of the compound of general formula [III] includes various derivatives usable in the so-called amidation reactions. Its concrete examples include isocyanate; isothiocyanates; derivatives formed by the reaction with silyl compounds such as trimethylsilylacetamide, bis(trimethylsilyl)acetamide and the like; derivatives formed by the reaction with aldehyde compounds such as acetaldehyde, isopentaldehyde, benzaldehyde,

salicylaldehyde, phenylacetaldehyde, p-nitrobenzaldehyde, m-chlorobenzaldehyde, hydroxynaphthaldehyde, furfural, thiophenecarboaldehyde and the like, hydrates of said aldehyde compounds or reactive derivatives such as acetals and the like, derivatives formed by the reaction with ketone compounds such as acetone, methyl ethyl ketone, methyl isobutyl ketone, acetylacetone, ethyl acetoacetate and the like or their reactive derivatives such as ketal, hemiketal, enolate and the like; reaction products with phosphorus compounds such as phosphorus oxychloride, phosphorus trichloride and the like; and reaction products with sulfur compounds such as thionyl chloride and the like.

As examples of the salt of compound represented by general formula [II], the same ones as mentioned above as examples of the salt of compound [I] can be referred to.

The reactive derivatives of the compounds represented by general formulas [II] and [III] are appropriately selected from the above-mentioned compounds depending on the kinds of starting compounds [II] and [III], other reactants and reaction conditions such as solvent, temperature, etc.

This reaction is usually carried out in a solvent having no adverse effect on the reaction, such as water, acetone, dioxane, acetonitrile, chloroform, benzene, methylene chloride, ethylene chloride, tetrahydrofuran, ethyl acetate, N,N-dimethylformamide, pyridine and the like or in a mixture of these solvents.

When the compound [II] is used in the form of a

free acid or its salt in this reaction, the reaction is preferably carried out in the presence of a condensing agent. Examples of said condensing agent include carbodiimide compounds such as N,N'-dicyclohexylcarbodiimide, N-cyclohexyl-N'-morpholinoethylcarbodiimide, N-cyclohexyl-N'-(4-diethylaminocyclohexyl)carbodiimide, N,N'-diethylcarbodiimide, N,N'-diisoprophylcarbodiimide, N-ethyl-N'-(3-dimethylaminopropyl)carbodiimide and the like; bis-imidazolide compounds such as N,N-carbonylbis(2-methylimidazole) and the like; 1-(4-chlorobenzenesulfonyloxy)-6-chloro-1H-benzotriazole; N-ethylbenzisoxazolium salt; N-ethyl-5-phenylisoxazolium-3'-sulfonate; phosphorus compounds such as polyphosphoric acid, trialkyl phosphite, ethyl polyphosphate, isopropyl polyphosphate, phosphorus oxychloride, phosphorus trichloride, diethyl chlorophosphate, o-phenylene chlorophophite and the like; and Vilsmeier reagent prepared by reacting dimethylformamide with thionyl chloride, phosphorus oxychloride, phosgen or the like.

The starting compound of general formula [II] can be produced by the known process or similar processes. The other starting compound represented by general formula [III] can be produced according to the following reaction:

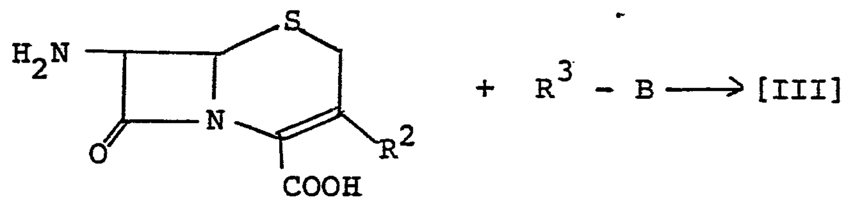

[IV] [V]

wherein B represents hydroxyl residue or its reactive derivative residue and $R^2$ and $R^3$ are as defined above. This reaction is practised by reacting an esterifying agent of general formula [V] upon the compound of general formula [IV], its derivative at its carboxyl residue or its salt in the presence or absence of a solvent having no adverse effect on the reaction, such as N,N-dimethylformamide, acetomide, dimethyl sulfoxide or the like, while cooling or heating the reaction system. When the hydroxy compound is liquid, it may be used simultaneously as a solvent.

As the reactive derivative of the compound of general formula [IV] at its carboxyl group or salt thereof, the same reactive derivatives as mentioned in the paragraph of salts and reactive derivatives of compound [II] can be referred to. In the compound represented by general formula [V], examples of the reactive derivative residue of the hydroxyl residue B include halogen atoms, alkylsulfonyloxy residues such as methanesulfonyloxy residue, ethanesulfonyloxy residue and the like, and arylsulfonyloxy residues such as benzenesulfonyloxy residue, p-toluenesulfonyloxy residue and the like.

In the above-mentioned reactions for producing the compounds represented by general formula [I] or [III], the starting compound [II] or [IV] may be reacted directly. Alternatively, when an amino group exists, it is also allowable to first protect the amino group with usual protecting group used in peptide syntheses, thereafter to react it, and then to remove the protecting group after the reaction by an appropriate method so as to yield the intended compound.

Process (b): Esterification

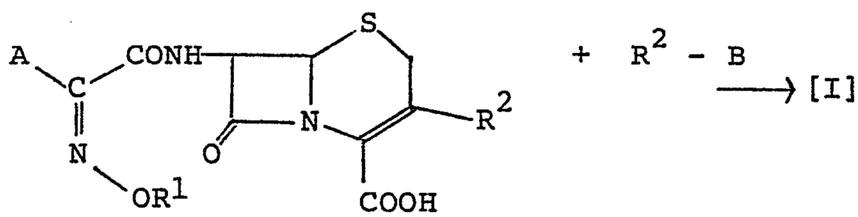

[VI] [V]

wherein A, B, $R^1$, $R^2$ and $R^3$ are as defined above.

The compound of general formula [I] can be produced by reacting a compound represented by general formula [VI], its reactive derivative at carboxyl residue or its salt, with an esterifying agent represented by general formula [V]. The reaction is carried out in the same manner as in the reaction for producing compound [III] by process (a), and the meanings of reactive derivative and reactive derivative residue at B are the same as above. In this reaction also, it is similarly allowable to first protect amino group, if it exists, with a protecting group usually used in peptide syntheses, then to

carry out the condensation reaction and thereafter to remove the protecting group. This reaction is carried out in the presence or absence of a solvent having no adverse effect on the reaction, while cooling or heating the reaction system. When the esterifying agents is liquid, it may be used simultaneously as a solvent.

Process (c): Oxime formation

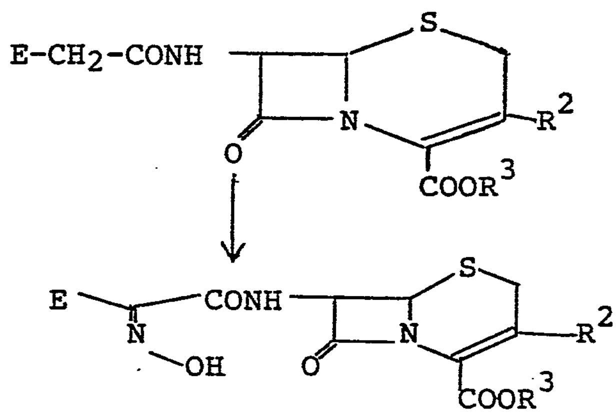

[VII]

[VIII]

wherein E represents haloacetyl residue, and $R^2$ and $R^3$ are as defined above.

The objective compound [VIII] is produced by reacting compound [VII] or its salt with a nitrosylating agent.

Preferable examples of the nitrosylating agent include nitrous acid and its derivatives, and more particularly nitrosyl halides such as nitrosyl chloride and nitrosyl bromide; alkali nitrites such as sodium nitrite and potassium nitrite; and alkyl nitrite, such as butyl nitrite and pentyl nitrite.

This reaction is usually carried out in a solvent. As the solvent, those having no adverse effect

on the reaction, such as water, acetic acid, benzene, methanol, ethanol, tetrahydrofuran and the like, can be used. Though the reaction temperature is not critical, it is usually preferable to carry out the reaction at a lowered or ambient temperature. The starting compound [VII] of this reaction can be produced by reacting compound [III] with a reactive derivative of $E-CH_2COOH$ or its salt. In practising this reaction, the same process as the above-mentioned process (a) or its analogous process can be employed. The compound [VII] can also be produced by reacting a compound represented by the following general formula:

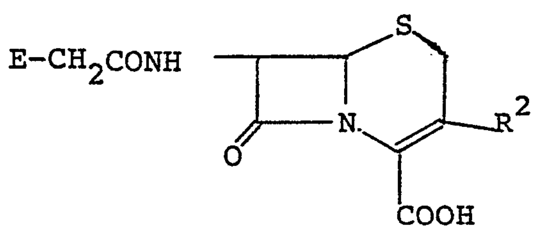

[IX]

wherein E and $R^2$ are as defined above, or its reactive derivative at carboxyl residue or its salt with an esterifying agent represented by the following general formula:

$$R^3- B$$

wherein B and $R^3$ are as defined above.

Process (d): Etherification

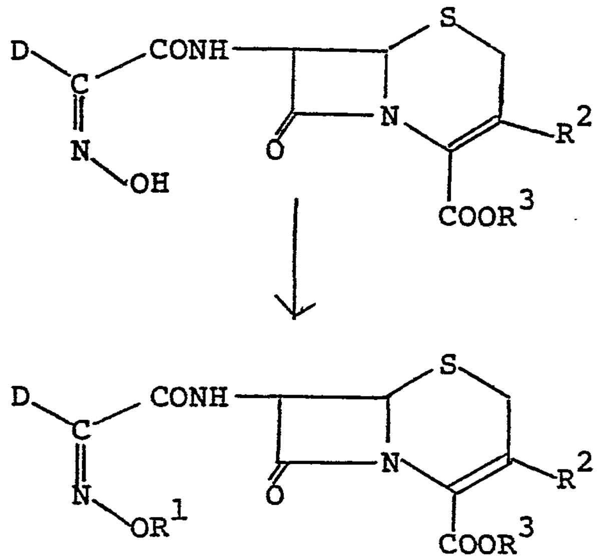

[X]

[XI]

wherein D represents a residue defined by the above-mentioned A or E, and $R^1$, $R^2$ and $R^3$ are as defined above.

The objective compound [XI] can be produced by reacting compound [X] or its salt with an etherifying agent. Preferable examples of the etherifying agent include usual alkylating agents such as dialkyl sulfate (e.g. dimethyl sulfate, diethyl sulfate and the like), diazoalkane (e.g. diazomethane, diazoethane and the like), alkyl halides (e.g. methyl iodide, ethyl iodide, ethyl bromide and the like), alkyl sulfonate (e.g. methyl toluenesulfonate and the like) and the like; as well as alkenylating, alkinylating and cycloalkylating agents corresponding to above; etherifying agents prepared by substituting the aliphatic hydrocarbon part of the above-mentioned alkylating agents with a carboxyl residue or an esterified carboxyl residue; halogenoalkenes such as

allyl iodide; halogenoalkines such as propargyl bromide; halogenocycloalkanes such as cyclohexyl bromide; and lower alkoxycarbonylalkyl halides such as ethoxycarbonylmethyl iodide. When a diazoalkane is used as the etherifying agent, it is usually preferable to carry out the reaction in a solvent having no adverse effect on the reaction, such as diethyl ether, dioxane or the like, at a lowered or ambient temperature.

Process (e): Thiazole ring formation

The objective compound represented by general formula [I] and its salt can be produced by reacting a compound represented by general formula [VIII] or [X] (when D is haloacetyl residue) or their salt with a compound represented by the following general formula:

$$R^4 - \overset{\overset{S}{\|}}{C} - NH_2$$

wherein $R^4$ represents hydrogen atom, halogen atom, lower alkyl, amino or acylamido.

This reaction is usually carried out in a solvent having no adverse effect on the reaction, such as water, alcohols (e.g. methanol and ethanol), benzene, toluene, ethyl acetate, dimethylformamide, tetrahydrofuran, dimethylsulfoxide and the like at ambient or elevated temperature.

Process (f): Other processes

The objective compound of the invention can also be produced by utilizing the interchange reaction

of the functional group of compound [I]. For example, the objective compound of the invention can be produced by utilizing an interchange reaction from hydroxymethyl to acyloxymethyl at $R^3$ or an interchange reaction from acetyloxymethyl to lower alkylthiomethyl, to thiadiazolylthiomethyl, to (lower alkyl)-substituted thiadizolylthiomethyl, to tetrazolylthiomethyl, or to (lower alkyl)-, (lower alkylaminoalkyl)- or carboxymethyl-substituted tetrazolylthiomethyl at $R^3$. These interchange reactions of functional group can be practised by the known process or similar processes.

For a therapeutic administration, the compound of general formula [I] may be used in the form of a pharmaceutical composition prepared by admixing it, as an active ingredient, with a pharmacologically acceptable carrier, such as inorganic or organic, solid or liquid diluent suitable for oral administration, for example. As such pharmaceutical compositions, capsule, tablet, sugar-coated tablet, ointment or suppository, solution, suspension, emulsion and the like can be referred to. If necessary, usual additives such as assistant, stabilizer, wetting agent, emulsifier, buffering agent and the like may be added to the composition. As such additives, lactose, magnesium stearate, clay, sucrose, corn starch, talc, stearic acid, gelatin, agar, pectin, peanut oil, olive oil, cacao butter, ethylene glycol and the like can be referred to.

Although the dosage of the compound of the

invention varies depending on the age and state of patient, the mean dosage of the compound of the invention exhibiting an effect on the treatment of bacterial infectious diseases is 10 mg to 1,000 mg per one administration. In general, the dosage for an adult per day is 1 mg to 5,000 mg and preferably 50 mg to 3,000 mg. Administration of a larger amount than above is also allowable.

The pharmacological effect of the compounds of the invention is as mentioned above. Its concrete values will be shown below by referrring to the cases of the compounds of examples mentioned later.

(1) The compound of the invention was suspended in 0.5% solution of methylcellulose and orally administered to mice* at a dose of 50 mg/kg. The excretion percentage of the parent compound (a compound of which 4-$COOR^3$ had been hydrolyzed) in the urine during the period of 0-24 hours after the administration was as follows, as measured by the bioassay using E. coli NIHJ.

Table 1

| Compound (Example No.) | Excretion percentage of parent compound in the urine of mice (0-24 hrs) (%) |
|---|---|
| 3 | 39 |
| 4 | 31 |
| 10 | 29 |
| 28 | 33 |
| 32 | 51 |
| 39 | 43 |
| 40 | 32 |
| 42 | 43 |
| Reference compound A** | 28 |

* ICR-slc male mice, 4 weeks old

** Reference compound A: Pivaloyloxymethyl 7-[2-(2-amino-4-thiazolyl)-2-methoxyiminoacetamide]-3-cephem-4-carboxylate (known compound)

1 (2) Stability (Half-life) of the compounds of the invention at 37°C in aqueous solutions having pH values of 7.4, 5.5 and 1.2 were as follows, as measured by HPLC.

Table 2

| Compound (Example No.) | Half-life (hr) | | |
|---|---|---|---|
| | pH 1.2 | pH 5.5 | pH 7.4 |
| 1 | >24 | >24 | >24 |
| 3 | >24 | >24 | >24 |
| 4 | >24 | >24 | 20.0 |
| 17 | >24 | >24 | 14.3 |
| 28 | >24 | >24 | 8.2 |
| 39 | >24 | >24 | >24 |
| 40 | >24 | >24 | >24 |
| 42 | >24 | >24 | >24 |
| 43 | >24 | >24 | >24 |
| 44 | >24 | >24 | 14.2 |
| Control compound A | >24 | >24 | 2.2 |

1 (3) MIC (μg/ml) of the compounds of the invention and the corresponding parent compounds (compounds of which 4-$COOR^3$ had been hydrolyzed) against E. Coli NIHJ JC-2 were measured. The results were as follows.

Table 3

| Compound (Example No.) | MIC of the compound of the invention (μg/ml) | MIC of the corresponding parent compound (μg/ml) |
|---|---|---|
| 1 | 0.78 | ≦0.013 |
| 3 | 0.78 | ≦0.013 |
| 4 | 0.39 | ≦0.013 |
| 10 | >200 | 3.13 |
| 17 | 0.39 | ≦0.013 |
| 28 | 0.10 | ≦0.013 |
| 31 | 12.5 | 0.05 |
| 32 | 0.20 | ≦0.013 |
| 39 | 3.13 | ≦0.013 |
| 40 | 3.13 | ≦0.013 |
| 42 | 1.56 | ≦0.013 |
| 43 | 0.78 | ≦0.013 |
| 44 | 3.13 | ≦0.013 |

Method of the experiment: Agar-dilution method (Inoculum size: $10^6$ cells/ml, modified Mühler-Hinton agar)

(4) Toxicity

The compounds of Examples 4, 20 and 42 were tested for acute toxicity on mice*. The results were as follows:

Table 4

| | $LD_{50}$ (mg/kg, p.o.) |
|---|---|
| Compound of Example 4 | >5,000 |
| Compound of Example 20 | >5,000 |
| Compound of Example 42 | >5,000 |

* Mice: ICR-slc male mice, 4 weeks old

Typical concrete examples of the objective compound obtained by the invention are as follows:

Allyl 7-[2-(2-amino-4-thiazolyl)-2-methoxyiminoacetamido]-3-cephem-4-carboxylate, 2-Methyl-2-propenyl 7-[2-(2-amino-4-thiazolyl)-2-methoxyiminoacetamido]-3-cephem-4-carboxylate, 2-Methyl-3-phenyl-2-propenyl 7-[2-(2-amino-4-thiazolyl)-2-methoxyiminoacetamido]-3-cephem-4-carboxylate, 2-Chlolo-2-propenyl 7-[2-methoxyimino-2-(4-thiazolyl)-acetamido]-3-(1-methyl-1H-tetrazol-5-yl)-thiomethyl-3-cephem-4-carboxylate, 2-Chloro-2-propenyl 7-[2-Methoxycarbonylmethoxyimino-2-(4-thazolyl)-acetamido]-3-(1-methyl-1H-tetrazol-5-yl)-thiomethyl-3-cephem-4-carboxylate, 2-Chloro-2-propenyl 7-(2-tert-butoxycarbonylmethoxyimino-2-(4-thiazolyl)-acetamido]-3-methyl-3-cephem-4-carboxylate, 2-Chloro-2-propenyl 7-[2-tert-butoxycarbonylmethoxyimino-2-(4-thiazolyl)-acetamido]-3-(1-methyl-1H-tetrazol-5-yl)-thiomethyl-3-cephem-4-carboxylate, 2-Chloro-2-propenyl 7-[2-carboxymethoxyimino-2-(4-thiazolyl)-acetamido]-3-(1-methyl-1H-tetrazol-5-yl)-thiomethyl-3-cephem-4-carboxylate, 2-Chloro-2-propenyl 7-[2-carboxymethoxyimino-2-(4-thiazolyl)-acetamido]-3-methyl-3-cephem-4-carboxylate, Allyl 7-[2-acetamido-4-thiazolyl)-2-methoxyimino-acetamido]-3-cephem-4-carboxylate, 2-Chloro-2-propenyl 7-[2-(2-amino-4-thiazolyl)-2-methoxyiminoacetamido]-3-(5-methyl-1,3,4-thiadiazol-2-ylthiomethyl)-3-cephem-4-carboxylate, 2-Chloro-2-propenyl 7-[2-(2-amino-4-thiazolyl)-2-methoxyiminoacetamido]-3-(1,3,4-thiadiazol-2-ylthiomethyl)-3-cephem-4-carboxylate, Crotyl 7-[2-(2-amino-4-thiazolyl)-2-methoxyiminoacetamido]-3-cephem-4-carboxylate, Cinnamyl 7-[2-(2-amino-4-thiazolyl)-2-methoxyiminoacetamido]-3-cephem-4-carboxylate 3-Chloro-2-propenyl 7-[2-(2-amino-4-thiazolyl)-2-methoxyiminoacetamido]-3-cephem-4-carboxylate, 2-Methyl-3-phenyl-2-propenyl 7-[2-(2-amino-4-thiazolyl)-2-methoxyiminoacetamido]-3-acetoxymethyl-3-cephem-4-carboxylate, 2-Methyl-3-acetoxymethyl-2-propenyl 7-[2-(2-amino-4-thiazolyl)-2-methoxyiminoacetamido]-3-cephem-4-carboxylate, 3-Methyl-2-butenyl 7-[2-(2-amino-4-thiazolyl)-2-methoxyiminoacetamido]-3-cephem-4-carboxylate, 2-Chloro-2-propenyl 7-[2-hydroxyimino-2-(2-methyl-4-thiazolyl)-acetamido]-3-(1-methyl-1H-tetrazol-5-yl)-thiomethyl-3-cephem-4-carboxylate, 2-Chloro-2-propenyl 7-[2-(2-bromo-4-thiazolyl)-2-methoxyimino-

acetamido]-3-(1-methyl-1H-tetrazol-5-yl)-thiomethyl-3-cephem-4-carboxylate, 2-Chloro-2-propenyl 7-[2-(2-amino-4-thiazolyl)-2-methoxyiminoacetamido]-3-(1-methyl-1H-tetrazol-5-yl)-thiomethyl-3-cephem-4-carboxylate, 3-Chloro-2-propenyl 7-[2-(2-amino-4-thiazolyl)-2-hydroxyiminoacetamido]-3-(1-methyl-1H-tetrazol-5-yl)-thiomethyl-3-cephem-4-carboxylate, 3-Methoxycarbonyl-2-propenyl 7-[2-(2-amino-4-thiazolyl)-2-methoxyiminoacetamido]-3-cephem-4-carboxylate, 2-Chloro-2-propenyl 7-[2-(2-amino-4-thiazolyl)-2-methoxyiminoacetamido]-3-methylthiomethyl-3-cephem-4-carboxylate, 1-Methyl-2-propenyl 7-[2-(2-amino-4-thiazolyl)-2-methoxyiminoacetamido]-3-cephem-4-carboxylate, 2-Chloro-2-propenyl 7-[2-(2-amino-4-thiazolyl)-2-methoxyiminoacetamido]-3-cephem-4-carboxylate, 4-Chloro-2-butynyl 7-[2-(2-amino-4-thiazolyl)-2-methoxyiminoacetamido]-3-cephem-4-carboxylate, Allyl 7-[2-(2-amino-4-thiazolyl)-2-methoxyiminoacetamido]-3-acetoxymethyl-3-cephem-4-carboxylate, and 2-Methyl-3-methoxyphenyl-2-propenyl 7-[2-(2-amino-4-thiazolyl)-2-methoxyiminoacetamido]-3-acetoxymethyl-3-cephem-4-carboxylate.

Further, the following esters of 7-[2-(2-amino-4-thiazolyl)-2-methoxyiminoacetamido]-3-cephem-4-carboxylic acid are also included in the typical concrete examples:

2-Propyl-3-phenyl-2-propenyl ester, 2-Chloro-3-phenyl-2-propenyl ester, 2-Methyl-3-tolyl-2-propenyl ester, 3-Tolyl-2-propenyl ester, 3-Methoxyphenyl-2-propenyl ester, 2-Methyl-3-methoxyphenyl-2-propenyl ester, 2-Ethyl-3-methoxyphenyl-2-propenyl ester, 2-Methyl-3-hydroxyphenyl-2-propenyl ester, 3-Hydroxyphenyl-2-propenyl ester, 2-Methyl-3-aminophenyl-2-propenyl ester, 3-Aminophenyl-2-propenyl ester, 2-Methyl-3-fluorophenyl-2-propenyl ester, and 2-Methyl-3-chlorophenyl-2-propenyl ester.

The invention will be illustrated more concretely by referring to the following examples.

Example 1

Allyl 7-[2-(2-amino-4-thiazolyl)-2-methoxyimino-acetamido]-3-cephem-4-carboxylate

( A: 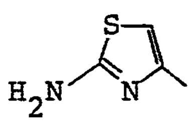 $R^1$: $CH_3$  $R^2$: H  $R^3$: $-CH_2CH=CH_2$ )

Allyl iodide (1.75 g) was added to 3.24 g of sodium 7-[2-(2-amino-4-thiazolyl)-2-methoxyiminoacetamido]-3-cephem-4-carboxylate stirred in 30 ml of dimethylformamide. After reacting them at room temperature for 5.5 hours, the reaction mixture was poured into a mixture consisting of 700 ml of ethyl acetate and 100 ml of aqueous hydrochloric acid having a pH value of 3.0, and thoroughly stirred. The organic layer was successively washed with 100 ml of aqueous hydrochloric acid having a pH value of 3.0, two 100 ml portions of phosphate buffer solution having a pH value

of 7.0 and 100 ml of saturated aqueous solution of sodium chloride, and then it was dried over anhydrous magnesium sulfate. It was filtered, concentrated under reduced pressure and then crystallized by dropping it in an ice-cooled ether. Filtration and washing with ether gave 2.71 g of the intended product.

PMR($CDCl_3$) δ(ppm): 3.5(2H,m) 4.01(3H,s), 4.74(2H,d), 5.02(1H,d), 5.15-6.0(3H,m), 6.05(1H,q), 6.55(1H,m), 6.72(1H,s), 7.90(1H,d)

IR: $1786 cm^{-1}$ (νc=0)

Melting point: 180 - 184°C

Example 2

Hydrochloride of the compound of Example 1

A 0.15 g portion of the allyl 7-[2-(2-amino-4-thiazolyl)-2-methoxyiminoacetamido]-3-cephem-4-carboxylate was dissolved into 10 ml of ethyl acete, into which was blown hydrogen chloride while cooling the solution with ice. After stirring it for 30 minutes, the resulting crystalline product was collected by filtration, washed with ethyl acetate and dried under reduced pressure to obtain 0.17 g of the intended product.

Example 3

2-Methyl-2-propenyl 7-[2-(2-amino-4-thiazolyl)-2-methoxyiminoacetamido]-3-cephem-4-carboxylate

( A: 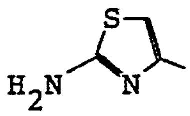  $R^1$: $CH_3$  $R^2$: H  $R^3$: 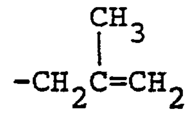 )

A mixture of 0.68 g of 3-chloro-2-methylpropene and 1.12 g of sodium iodide was heated under reflux in 10 ml of acetone for 30 minutes. The resulting crystalline product was collected by filtration, washed with a small quantity of methylene chloride and united with the acetone solution. The united mixture was carefully distilled to remove methylene chloride and acetone. The residue was added to a stirred mixture of 2.03 g of sodium 7-[2-(2-amino-4-thiazolyl)-2-methoxyiminoacetamido]-3-cephem-4-carboxylate and 20 ml of dimethylformamide, after which it was reacted at room temperature for 12 hours. Subsequently, the same treatment as in Example 1 gave 1.8 g of the intended product.

PMR($CDCl_3$) δ(ppm): 1.77(3H,s), 3.52(2H,m), 4.01(3H,s), 4.63(2H,s), 4.9-5.1(3H,m), 5.6(2H,broad s), 6.06(1H,q), 6.58(1H,m), 6.74(1H,s), 7.82(1H,d)

IR: 1784$cm^{-1}$ (νc=0)

Melting point: 123 - 133°C

Example 4

2-Methyl-3-phenyl-2-propenyl 7-[2-(2-amino-4-thiazolyl)-2-methoxyiminoacetamido]-3-cephem-4-carboxylate

( A: 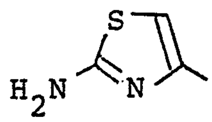  $R^1$: $CH_3$  $R^2$: H

$R^3$: 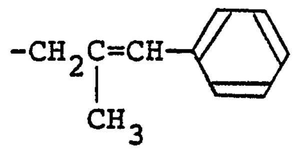 )

Into an ice-cooled and stirred mixture consisting of 3 ml of ethyl acetate and 0.17 g of dimethylformamide was dropwise added 0.23 g of phosphrus oxychloride. After stirring the resulting mixture for 1.5 hours while cooling it with ice, 0.23 g of 2-(2-formamido-4-thiazolyl)-2-methoxyiminoacetic acid (syn isomer) was added, and the whole mixture was stirred for an additional one hour. The resulting solution was dropwise added to a stirred solution consisting of 8 ml of ethyl acetate, 2 ml of pyridine and 0.33 g of 2-methyl-3-phenyl-2-propenyl 7-amino-3-cephem-4-carboxylate at -50°C, and stirred for one hour. Subsequently, the reaction mixture was slowly warmed to 0°C, poured into a mixture of 80 ml of ethyl acetate and 20 ml of dilute hydrochloric acid, and stirred thoroughly. After separating the mixture into layers, the organic layer was washed successively with dilute hydrochloric acid, phosphate buffer solution having a pH value of 7.0 and saturated aqueous solution of sodium chloride, and dried over anhydrous magnesium sulfate. After filtration and concentration under reduced pressure, the concentrate was dropped into an ice-cooled ether. The resulting

powder was collected by filtration, washed with ether and dried under reduced pressure. Then, the powder was added to an ice-cooled mixture consisting of 6 ml of methanol, 6 ml of tetrahydrofuran and 0.08 ml of concentrated hydrochloric acid, and stirred for 5 hours.

The reaction mixture was poured into a mixture of 150 ml of ethyl acetate and 30 ml of phosphate buffer solution having a pH value of 7.0. After separation into layers, the organic layer was washed successively with a phosphate buffer solution having a pH value of 7.0 and a saturated aqueous solution of sodium chloride and dried over anhydrous magnesium sulfate. After filtration and concentration under reduced pressure, the concentrate was dropped into ice-cooled ether, and the resulting crystalline product was collected by filtration and washed with ether to obtain 0.46 g of the intended product.

PMR($CDC\ell_3$) δ(ppm): 1.90(3H,m), 3.47(2H,m), 3.98(3H,s), 4.80(2H,m), 5.00(1H,d), 6.03(1H,q), 6.50(2H,broad s), 6.68(1H,s), 7.21(5H,m), 7.85(1H,q)

IR: $1785cm^{-1}$ (νC=0)

Melting point: 95 - 105°C

Referential Example 1

Synthesis of 2-chloro-3-iodo-1-propene

One hundred grams of 2,3-dichloro-1-propene and 135 g of sodium iodide were heated for 30 minutes under reflux in 300 ml of acetone. The resulting

crystal was filtered off, and the solvent was distilled off under ordinary pressure. Distillation of the residue under a reduced pressure gave 140 g of the intended product.

PMR($CCl_4$) δ(ppm): 4.0(2H,s), 5.3(1H,s), 5.6(1H,s)

Boiling point: 60°C (33 mmHg)

Example 5

2-Chloro-2-propenyl 7-[2-methoxyimino-2-(4-thiazolyl)-acetamido]-3-(1-methyl-1H-tetrazol-5-yl)-thiomethyl-3-cephem-4-carboxylate

A: 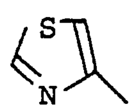 $R^1$: $CH_3$ $R^2$: 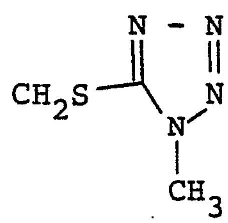

$R^3$: 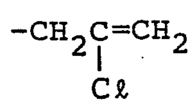

To 1.05 g of sodium 7-[2-methoxyimino-2-(4-thiazolyl)-acetamido]-3-(1-methyl-1H-tetrazol-5-yl)-thiomethyl-3-cephem-4-carboxylate stirred in 10 ml of dimethylformamide, 420 mg of 2-chloro-3-iodo-1-propene was added. They were reacted at room temperature for 5 hours. Subsequently, the same treatment as in Example 1 gave 0.81 g of the intended product.

PMR($CDCl_3$) δ(ppm): 3.80(2H,s), 3.88(3H,s), 4.08(3H,s), 4.4(2H,q), 4.7-4.9(2H,m), 5.08(1H,d),

5.3-5.7(2H,m), 5.9-6.1(1H,m), 7.78(1H,s), 8.78(1H,s)

IR: 1792 $cm^{-1}$ ($\nu_{C=O}$)

Melting point: 91 - 94°C

Example 6

2-Chloro-2-propenyl 7-[2-methoxycarbonylmethoxyimino-2-(4-thiazolyl)-acetamido]-3-(1-methyl-1H-tetrazol-5-yl)-thiomethyl-3-cephem-4-carboxylate

A: 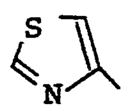 $R^1$: $-CH_2CO_2CH_3$, $R^2$: $-CH_2S$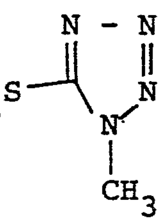

$R^3$: 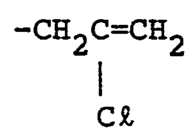

The intended product was synthesized from sodium 7-[2-methoxycarbonylmethoxyimino-2-(4-thiazolyl)-acetamido]-3-(1-methyl-1H-tetrazol-5-yl)-thiomethyl-3-cephem-4-carboxylate and 2-chloro-3-iodo-1-propene in the same manner as in Example 5.

PMR($d_6$-DMSO) δ(ppm): 3.65(3H,s), 3.7(2H,m), 3.90(3H,s), 4.10-4.50(2H,q), 4.73(2H,s), 4.82(2H,s), 5.17(1H,d), 5.50(1H,m) 5.7(1H,m), 5.78(1H,q), 7.90(1H,d), 8.10(1H,d), 9.64(1H,d)

IR: 1786$cm^{-1}$($\nu$C=0)

Melting point: 85 - 95°C

Example 7

2-Chloro-2-propenyl 7-[2-tert-butoxycarbonylmethoxyimino-2-(4-thiazolyl)-acetamido]-3-methyl-3-cephem-4-carboxylate

A: 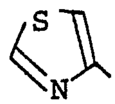 $R^1$: $-CH_2CO_2C(CH_3)_3$ , $R^2$: $CH_3$ ,

$R^3$: $-CH_2\underset{Cl}{\underset{|}{C}}=CH_2$

The intended product was synthesized by reacting 7-[2-tert-butoxycarbonylmethoxyimino-2-(4-thiazolyl)-acetamido]-3-cephem-4-carboxylic acid and 2-chloro-3-iodo-1-propene in the same manner as in Example 5 to obtain the product in the form of a solution in ethyl acetate, followed by distilling off the solvent under a reduced pressure.

PMR($d_6$-DMSO) δ(ppm): 1.42(9H,s), 2.03(3H,s), 3.2-3.8(2H,q), 4.60(2H,s), 4.82(2H,s), 5.16(1H,d), 5.48(1H,m), 5.72(1H,m), 5.80(1H,q), 7.90(1H,d), 9.10(1H,d), 9.55(1H,q)

IR: 1788$cm^{-1}$ (νC=0)

Example 8

2-Chloro-2-propenyl 7-[2-tert-butoxycarbonylmethoxyimino-2-(4-thiazolyl)-acetamido]-3-(1-methyl-1H-tetrazol-5-yl)-thiomethyl-3-cephem-4-carboxylate

A: 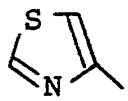 , $R^1$: $-CH_2CO_2{}^tBu$, $R^2$: $-CH_2-S$ 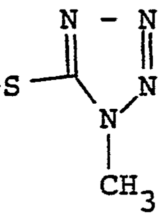

$R^3$: 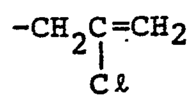

One hundred milligrams of sodium 7-[2-tert-butoxycarbonylmethoxyimino-2-(4-thiazolyl)-acetamido]-3-(1-methyl-1H-tetrazol-5-yl)-thiomethyl-3-cephen-4-carboxylate was stirred in 1 ml of dimethylformamide, to which was added 34.9 mg of 2-chloro-3-iode-1-propene. The mixture was reacted at room temperature for 6 hours. Then, the reaction mixture was treated in the same manner as in Example 1 to obtain 95.0 mg of the intended product.

PMR($CDC\ell_3$) δ(ppm): 1.39(9H,s), 3.71(2H,s), 3.88(3H,s), 4.05-4.4(2H,m), 4.6-4.8(4H,m), 5.05(1H,d), 5.4-5.6(2H,m), 5.8-6.02(1H,m), 7.76(1H,s), 8.5-8.7(1H,m), 8.79(1H,s)

IR: $1797cm^{-1}$ ($\nu$C=0)

Melting point: 63 - 65°C

Example 9

2-Chloro-2-propenyl 7-[2-carboxymethoxyimino-2-(4-thiazolyl)-acetamido]-3-(1-methyl-1H-tetrazol-5-yl)-thiomethyl-3-cephem-4-carboxylate

A: 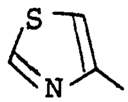 $R^1$: $-CH_2CO_2H$ , $R^2$: $-CH_2-S-$ 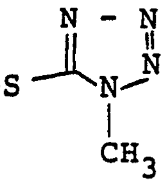

$R^3$: 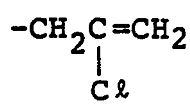

Into 0.5 ml of trifluoroacetic acid were dissolved 54.6 mg of 2-chloro-2-propenyl 7-[2-tert-butoxycarbonylmethoxyimino-2-(4-thiazolyl)-acetamido]-3-(1-methyl-1H-tetrazol-5-yl)-thiomethyl-3-cephem-4-carboxylate and 0.1 ml of anisole, and the resulting solution was stirred at room temperature for 2 hours. Then, the solvent was distilled off under reduced pressure. The residue was mixed with 2 ml of water and extracted with 10 ml of ethyl acetate. The extract was washed with three 5 ml portions of water and dried over magnesium sulfate. After distilling off the solvent under reduced pressure, the residue was treated with diethyl ether to obtain 35.0 mg of the intended product as a powdery substance.

PMR($CDCl_3$) δ(ppm): 3.74(2H,d), 3.96(3H,d), 4.3(2H,q), 4.7-4.9(4H,m), 5.08(1H,d), 5.3-5.6(2H,m), 5.8-6.1(1H,m), 7.8(1H,m), 8.8(1H,m)

IR: $1793cm^{-1}$ ($\nu$C=0)

Melting point: 86 - 88°C

Example 10

2-Chloro-2-propenyl 7-[2-carboxymethoxyimino-2-(4-thiazolyl)-acetamido]-3-methyl-3-cephem-4-carboxylate

( A: 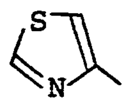 , $R^1$: $-CH_2CO_2H$ , $R^2$: $CH_3-$ , $R^3$: $-CH_2\underset{|\atop Cl}{C}=CH_2$ )

The intended product was synthesized by treating 7-[2-tert-butoxycarbonylmethoxyimino-2-(4-thiazolyl)-acetamido]-3-methyl-3-cephem-4-carboxylic acid in the same manner as in Example 9.

PMR($d_6$-DMSO) δ(ppm): 2.04(3H,s), 3.05-3.73 (2H,q), 4.65(2H,s), 4.83(2H,s), 5.17(1H,d), 5.49(1H,m), 5.73(1H,m), 5.80(1H,q), 7.93(1H,d), 9.12(1H,d), 9.56 (1H,d)

IR: 1786$cm^{-1}$ (νC=0)

Melting point: 125 - 135°C

Example 11

Allyl 7-[2-(2-acetylamido-4-thiazolyl)-2-methoxyiminoacetamido]-3-cephem-4-carboxylate

( A: 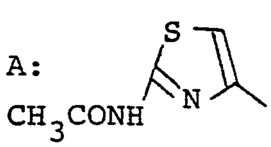 , $R^1$: $CH_3$ , $R^2$: H , $R^3$: $-CH_2CH=CH_2$ )

Allyl 7-[2-(2-amino-4-thiazolyl)-2-methoxyimino-acetamido]-3-cephem-4-carboxylate (210 mg) was dissolved into 20 ml of acetonitrile and cooled to -40°C. After adding 120 mg of dimethylaniline, 0.08 ml of acetyl chloride was added, and temperature of the mixture was raised to 0°C. After reacting the mixture at this temperature for 50 hours, it was after-treated in the same manner as in Example 1. The solution ethyl acetate thus obtained was concentrated under a reduced pressure, the concentrate was deopped into cyclohexane to form a powdery product, the powder was washed with a small quantity of ethyl acetate, and then it was filtered and dried. Thus, the intended product was obtained.

PMR($d_6$-DMSO) $\delta$(ppm): 2.12(3H,s), 3.60(2H,m), 3.87(3H,s), 4.70(2H,m), 5.12(1H,d), 5.27(1H,m), 5.43 (1H,m), 5.6-6.1(2H,m), 6.57(1H,m), 7.29(1H,s), 9.65 (1H,d), 12.42(1H,s)

IR: 1778$cm^{-1}$($\nu$C=0)

Melting point: Higher than 180°C

Example 12

2-Chloro-2-propenyl 7-[2-(2-amino-4-thiazolyl)-2-methoxyiminoacetamido]-3-(5-methyl-1,3,4-thiadiazol-2-ylthiomethyl)-3-cephem-4-carboxylate

A: 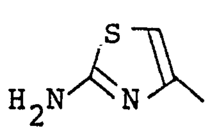 , $R^1$: $CH_3$ , $R^2$: 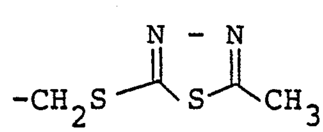

$R^3$: 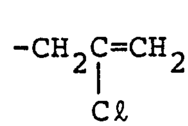

The intended product was synthesized in the same manner as in Example 7.

PMR($d_6$-DMSO) δ(ppm): 2.65(3H,s), 3.5-3.9 (2H,q), 3.80(3H,s), 4.0-4.7(2H,q), 4.82(2H,m), 5.16 (1H,d), 5.50(1H,m), 5.6-5.9(2H,m), 6.68(1H,s), 7.15 (2H,s), 9.55(1H,d)

IR: 1786$cm^{-1}$ (νC=O)

Melting point: 120 - 140°C

Example 13

2-Chloro-2-propenyl 7-[2-(2-amino-4-thiazolyl)-2-methoxyimino-acetamido]-3-(1,3,4-thiadiazol-2-ylthiomethyl)-3-cephem-4-carboxylate

A: 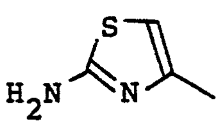 , $R^1$: $CH_3$ , $R^2$: $-CH_2S$ 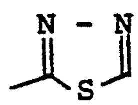 ,

$R^3$: 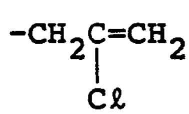

The intended product was produced in the same manner as in Example 5.

PMR($d_6$-DMSO) δ(ppm): 3.4-4.0(2H,q), 3.80 (3H,s), 4.1-4.7(2H,q), 4.83(2H,m), 5.80(1H,d), 5.48 (1H,m), 5.6-5.9(2H,m), 6.68(1H,s), 8.14(2H,s), 9.48 (1H,s), 9.54(1H,d)

IR: 1788$cm^{-1}$ (νC=O)

Melting point: 120 - 133°C

Example 14

Crotyl 7-[2-(2-amino-4-thiazolyl)-2-methoxyiminoacetamido]-3-cephem-4-carboxylate

( A: 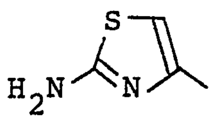 , $R^1$: $CH_3$ , $R^2$: H , $R^3$: $-CH_2CH=CHCH_2$ )

The intended produce was produced by repeating the procedure of Example 3, except that the 3-chloro-2-methylpropene was replaced with crotyl chloride.

PMR($CDCl_3$) δ(ppm): 1.72(2H,d), 3.50(2H,m), 4.00(3H,s), 4.5-4.9(2H,m), 5.02(1H,d), 5.4-6.1(4H,m), 6.03(1H,q), 6.53(1H,m), 6.72(1H,s), 7.90(1H,d)

IR: 1786$cm^{-1}$ ($\nu$C=0)

Melting point: 108-115°C

Example 15

Cinnamyl 7-[2-(2-amino-4-thiazolyl)-2-methoxyiminoacetamido]-3-cephem-4-carboxylate

( A: 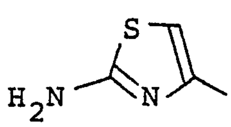 , $R^1$: $CH_3$, $R^2$: H , $R^3$: $-CH_2CH=CH-$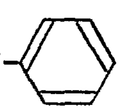 )

The intended product was produced by repeating the procedure of Example 3, except that the 3-chloro-2-methylpropene was replaced with cinnamyl chloride.

PMR($CDCl_3$) δ(ppm); 3.50(2H,m), 4.00(3H,s), 4.6-5.1(2H,m), 5.03(1H,d), 5.75(2H,s), 5.9-6.9(5H,m),

7.0-7.6(5H,m), 7.95(1H,d)

IR: $1786cm^{-1}$ ($\nu$C=O)

Melting point: 150 - 155°C

Example 16

2-Methyl-3-phenyl-2-propenyl 7-[2-(2-amino-4-thiazolyl)-2-methoxyiminoacetamido]-3-cephem-4-carboxylate

( A: 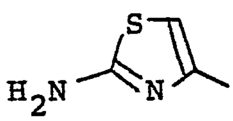 , $R^1$: $CH_3$ , $R^2$: H , $R^3$: 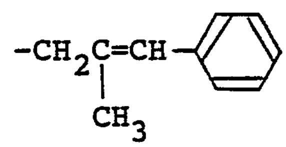 )

In 13 ml of acetone, 1.30 g of 1-chloro-2-methyl-3-phenyl-2-propene and 1.17 g of sodium iodide were heated under reflux for 30 minutes. The resulting crystal was filtered and washed with a small quantity of acetone. The filtrate was united with the washings, from which the acetone was distilled off. The residue was added to a stirred mixture of 3.16 g of sodium 7-[2-(2-amino-4-thiazolyl)-2-methoxyiminoacetamido]-3-cephem-4-carboxylate and 32 ml of dimethylformamide, and reacted at room temperature for 3 hours. Thereafter, the reaction mixture was treated in the same manner as in Example 1 to form 2.53 g of the intended product.

Its PMR, IR and melting point were all the same as those of the product of Example 4.

Example 17

3-Chloro-2-propenyl 7-[2-(2-amino-4-thiazolyl)-2-methoxyiminoacetamido]-3-cephem-4-carboxylate

( A: 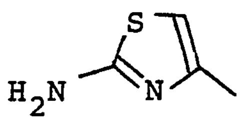 , $R^1$: $CH_3$ , $R^2$: H , $R^3$: $-CH_2CH=CHCl$ )

In 27 ml of acetone, 2.77 g of 1,3-dichloro-1-propene and 3.75 g of sodium iodide were heated under reflux for 30 minutes. The resulting crystal was filtered and washed with a small quantity of acetone. The filtrate and the washings were united, from which the acetone was distilled off carefully. The residue was added to a stirred mixture of 4.05 g of sedium 7-[2-(2-amino-4-thiazolyl)-2-methoxyiminoacetamido]-3-cephem-4-carboxylate and 40 ml of dimethylformamide, and reacted at room temperature for 5 hours. Then, the reaction mixture was treated in the same manner as in Example 1 to form 3.6 g of the intended product.

PMR($CDCl_3$) $\delta$(ppm): 3.35-3.63(2H,m), 3.99 (3H,s), 4.81(2H,m), 5.02(1H,d), 5.79-6.36(3H,m), 6.41-6.64(1H,m), 6.70(1H,s), 7.84(1H,d)

IR: 1785$cm^{-1}$($\nu$C=0)

Melting point: 132 - 138°C

Example 18

2-Meltyl-3-phenyl-2-propenyl 7-[2-(2-amino-4-thiazolyl)-2-methoxyiminoacetamido]-3-acetoxymethyl-3-cephem-4-carboxylate

A: 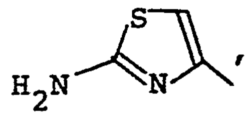, $R^1$: $CH_3$ , $R^2$: $CH_2OCOCH_3$ ,

$R^3$: 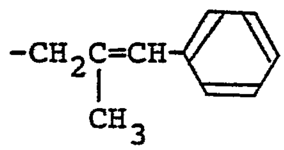

In 10.8 ml of acetone, 1.08 g of 1-chloro-2-methyl-3-phenyl-2-propene and 0.98 g of sodium iodide were heated under reflux for 30 minutes. The resulting crystal was filtered and washed with a small quantity of acetone. The filtrate and the washings were united, from which the acetone was distilled off. The residue was added to a stirred mixture of 2.39 g of sodium 7-[2-(2-amino-4-thiazolyl)-2-methoxyiminoacetamido]-3-acetoxymethyl-3-cephem-4-carboxylate and 24 ml of dimethylformamide, and reacted at room temperature for 3 hours. Then, the reaction mixture was treated in the same manner as in Example 1 to form 2.20 g of the intended product.

PMR($CDCl_3$) δ(ppm): 1.96(3H,m), 2.06(3H,s), 3.20-3.72(2H,q), 4.03(3H,s), 4.64-5.30(5H,m), 6.06 (1H,q), 6.56(1H, broad s), 6.70(1H,s), 7.25(5H,m), 7.95(1H,d)

IR: $1790cm^{-1}$

Melting point: 115 - 133°C

Example 19

2-Methyl-3-acetoxymethyl-2-propenyl 7-[2-(2-amino-4-thiazolyl)-2-methoxyiminoacetamido]-3-cephem-4-carboxylate

( A: 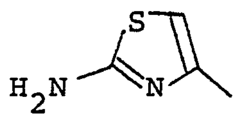 , $R^1$: $CH_3$ , $R^2$: H , $R^3$: $-CH_2\underset{CH_3}{\underset{|}{C}}=CHCH_2OCOCH_3$ )

In 10.6 ml of acetone, 1.06 g of 1-chloro-2-methyl-3-acetoxymethyl-2-propene and 0.98 g of sodium iodide were heated under reflux for 30 minutes. The resulting crystal was filtered and washed with a small quantity of acetone. The filtrate and the washings were united, from which the acetone was distilled off. The residue was added to a stirred mixture of 2.02 g of sodium 7-[2-(2-amino-4-thiazolyl)-2-methoxyiminoacetamide]-3-cephem-4-carboxylate and 20 ml of dimethylformamide, and reacted at room temperature for 12 hours. Then, the reaction mixture was treated in the same manner as in Example 1 to form 1.80 g of the intended porduct.

PMR($CDCl_3$) $\delta$(ppm): 1.80(3H,m), 2.04(3H,m), 3.50(2H,m), 4.01(3H,s), 4.41-4.87(4H,m), 5.05(1H,d), 5.60(1H,m), 6.07(1H,q), 6.47(1H,m), 6.69(1H,s), 8.00 (1H,d)

IR: 1790 $cm^{-1}$

Melting point: 112 - 128°C

Example 20

3-Methyl-2-butenyl 7-[2-(2-amino-4-thiazolyl)-2-methoxyiminoacetamido]-3-cephem-4-carboxylate

( A: 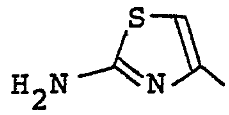 , $R^1$: $CH_3$ , $R^2$: H , $R^3$: 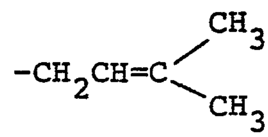 )

In 20 ml of acetone, 0.78 g of 4-chloro-2-methyl-2-butene and 1.12 g of sodium iodide were heated under reflux for 30 minutes. The resulting crystal was filtered and washed with a small quantity of methylene chloride. The filtrate and the washings were united, from which the methylene chloride and acetone were distilled off at ordinary pressure. The residue was added to a stirred mixture of 2.02 g of sodium 7-[2-(2-amino-4-thiazolyl)-2-methoxyiminoacetamido]-3-cephem-4-carboxylate and 20 ml of dimethylformamide and reacted at room temperature for 5.5 hours. Then, the reaction mixture was treated in the same manner as in Example 1 to form 1.50 g of the intended product.

PMR($CDCl_3$) $\delta$(ppm): 1.72(6H,d), 3.49(2H,d), 4.04(3H,s), 4.73(2H,d), 5.04(1H,d), 5.2-5.6(3H,m), 6.0(1H,q), 6.5(1H,q), 6.80(1H,s), 7.60(1H,d)

IR: 1785$cm^{-1}$($\nu$C=0)

Melting point: 148 - 150°C

Example 21

2-Chloro-2-propenyl 7-[2-hydroxyimino-2-(2-methyl-4-thiazolyl)-acetamido]-3-(1-methyl-1H-tetrazol-5-yl)-thiomethyl-3-cephem-4-carboxylate

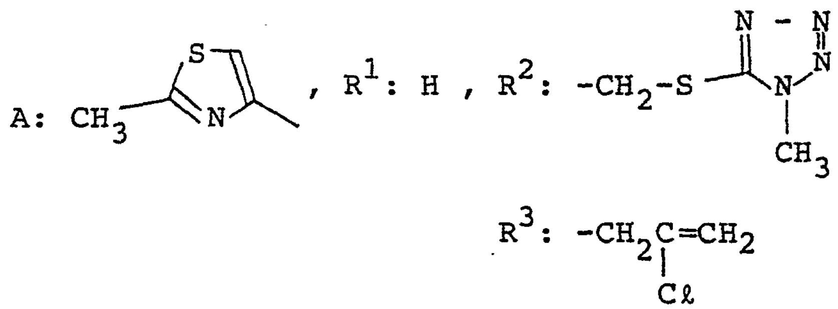

To a stirred mixture consisting of 78.6 mg of 7-[2-hydroxyimino-2-(2-methyl-4-thiazolyl)-acetamido]-3-(1-methyl-1H-tetrazol-5-yl)-thiomethyl-3-cephem-4-carboxylic acid and 0.5 ml of dimethylformamide, 33 mg of 2-chloro-3-iodo-1-propene and 16 mg of triethylamine were added. The mixture was reacted at room temperature for 8 hours. Then, the reaction mixture was treated in the same manner as in Example 1 to form 56 mg of the intended product.

PMR($CDC\ell_3$) δ(ppm): 2.71(3H,s), 3.81(2H,s), 3.91(3H,s), 4.44(2H,d), 4.85(2H,s), 5.08(1H,d), 5.4-5.6 (2H,m), 5.9(1H,q), 7.67(1H,s)

IR: $1792 cm^{-1}$ ($\nu C=0$)

Melting point: 81.- 84°C

Example 22

2-Chloro-2-propenyl 7-[2-(2-bromo-4-thiazolyl)-2-methoxyiminoacetamido]-3-(1-methyl-1H-tetrazol-5-yl)-thiomethyl-3-cephem-4-carboxylate

A: 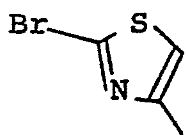 , $R^1$: $CH_3$ , $R^2$: 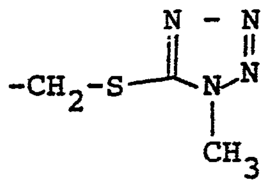

$R^3$: 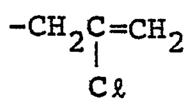

Seventy milligrams of 2-chloro-3-iodo-1-propene was added to a stirred mixture consisting of 134 mg of sodium 7-[2-(2-bromo-4-thiazolyl)-2-methoxyiminoacetamido]-3-(1-methyl-1H-tetrazol-5-yl)-thiomethyl-3-cephem-4-carboxylate and 1 ml of dimethylformamide. After reacting the mixture at room temperature for 4 hours, it was treated in the same manner as in Example 1 to form 78 mg of the intended product.

PMR($CDCl_3$) δ(ppm): 3.79(2H,s), 3.90(3H,s), 4.09(3H,s), 4,38(2H,d), 4.84(2H,s), 5.06(1H,d), 5.4-5.6(2H,m), 6.0(1H,q), 7.68(1H,s)

IR: 1794$cm^{-1}$ ($\nu$C=0)

Melting point: 94 - 98°C

Example 23

2-Chloro-2-propenyl 7-[2-(2-amino-4-thiazolyl)-2-methoxyiminoacetamido]-3-(1-methyl-1H-tetrazol-5-yl)-thiomethyl-3-cephem-4-carboxylate

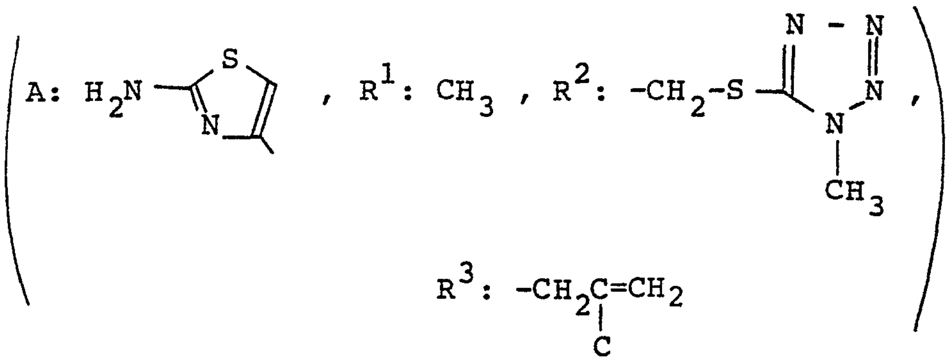

Sixty nine milligrams of 2-chloro-3-iodopropene was added to a stirred mixture consisting of 216 mg of sodium 7-[2-(2-amino-4-thiazolyl)-2-methoxyiminoacetamido]-3-(1-methyl-1H-tetrazol-5-yl)-thiomethyl-3-cephem-4-carboxylate and 1 ml of dimethylformamide, and reacted at room temperature for 5 hours. Then the reaction mixture was treated in the same manner as in Example 1 to form 152 mg of the intended product.

PMR ($CDCl_3$) δ (ppm): 3.7-4.1 (8H,m), 4.38 (2H,d), 4.6-4.9 (2H,m), 5.06 (1H,d), 5.2-5.6 (4H,m), 5.9-6.1 (1H,m), 6.76 (1H,s), 7.54 (1H,d)

IR: $1785 cm^{-1}$ ($\nu_{C=O}$)

Melting point: 108 - 115°C

Example 24

3-Chloro-2-propenyl 7-[2-(2-amino-4-thiazolyl)-2-hydroxyiminoacetamido]-3-(1-methyl-1H-tetrazol-5-yl)-thiomethyl-3-cephem-4-carboxylate

(A: $NH_2$ 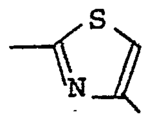 , $R^1$: H, $R^2$: $-CH_2-S$ 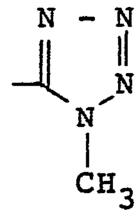

$R^3$: $-CH_2CH=CHCl$)

In 20 ml of acetone, 0.83 g of 1,3-dichloropropene and 1.12 g of sodium iodide were heated under reflux for 30 minutes. The resulting crystal was filtered and washed with a small quantity of methylene chloride. The filtrate and the washings were united, from which the methylene chloride and acetone were distilled off. The residue was added to a stirred mixture of 2.68 g of 7-[2-(2-amino-4-thiazolyl)-2-hydroxyiminoacetamido]-3-(1-methyl-1H-tetrazol-5-yl)-thiomethyl-3-cephem-4-carboxylic acid, 1.00 g of triethylamine and 20 ml of dimethylformamide, and reacted at room temperature for 20 hours. To the reaction mixture were added 20 ml of aqueous hydrochloric acid having a pH value of 3 and 20 ml of ethyl acetate. The resulting crystal was washed with ethyl acetate and metanol, and there was obtained 0.98 g of the intended product.

PMR(DMSO-$d_6$) δ(ppm): 3.7(2H,m), 3.90(3H,s), 4.1-4.5(2H,m), 4.6-5.0(2H,m), 5.0-5.2(1H,m), 6.0-6.2 (1H,m), 6.5-6.7(2H,m), 7.07(1H,s), 9.40(1H,d), 5.7-5.9 (1H,m)

IR: $1788cm^{-1}$ ($\nu C=O$)

Melting point: 184 - 188°C

Example 25

3-Methoxycarbonyl-2-propenyl 7-[2-(2-amino-4-thiazolyl)-2-methoxyiminoacetamido]-3-cephem-4-carboxylate hydrochloride.

(A: 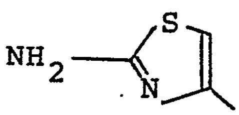 , $R^1$: $CH_3$ , $R^2$: H , $R^3$: $-CH_2CH=CHCO_2CH_3$)

In 20 ml of acetone, 1.58 g of methyl 4-bromo-2-butanoate and 1.12 g of sodium iodide were heated under reflux for 30 minutes. The resulting crystal was filtered and washed with a small quantity of methylene chloride, and the washings were united with the filtrate. The methylene chloride and acetone were distilled off at ordinary pressure. The residue was added to a stirred mixture consisting of 2.02 g of sodium 7-[2-(2-amino-4-thiazolyl)-2-methoxyiminoacetamido]-3-cephem-4-carboxylate and 20 ml of dimethylformamide, and reacted at room temperature for 6 hours. The reaction mixture was poured into a mixture consisting of 400 ml of ethyl acetate and 70 ml of aqueous hydrochloric acid having a pH value of 3.0, and stirred thoroughly. The organic layer was washed successively with two 70 ml portions of a phosphate buffer solution having a pH value of 7.0 and two 70 ml portions of saturated aqueous solution of sodium chloride, dried over

magnesium sulfate, filtered, and then concentrated under reduced pressure to a volume of about 100 ml. Gaseous hydrogen chloride was introduced into the concentrate, and the resulting crystalline product was collected by filtration. Thus, 1.60 g of the intended product was obtained.

PMR ($CDCl_3$) δ (ppm): 3.4-4.4 (8H,m), 4.8-5.2 (3H,m), 5.8-6.0 (1H,m), 6.07 (1H,d), 6.60 (1H,m), 6.8-7.1 (1H,m), 7.12 (1H,s), 8.60 (1H,d)

IR: $1784cm^{-1}$ ($\nu$C=0)

Melting point: 156 - 158°C

Example 26

2-Chloro-2-propenyl 7-[2-(2-amino-4-thiazolyl)-2-methoxyiminoacetamido]-3-methylthiomethyl-3-cephem-4-carboxylate

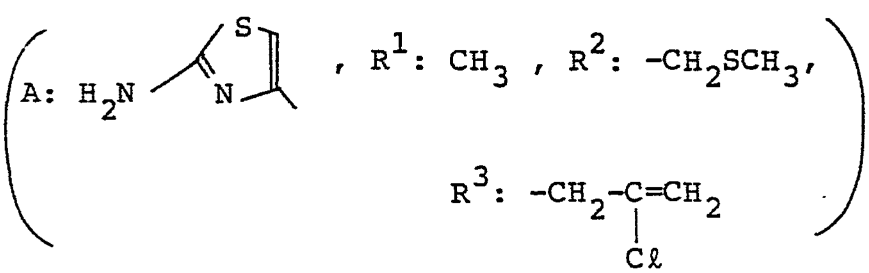

2-Chloro-3-iodo-1-propene (1.32 g) was added to a stirred mixture consisting of 2.44 g of sodium 7-[2-(2-amino-4-thiazolyl)-2-methoxyiminoacetamido]-3-methylthiomethyl-3-cephem-4-carboxylate and 25 ml of dimethylformamide, and reacted at room temperature for 3 hours.

Then, the reaction mixture was treated in the same manner as in Example 1 to form 2.1 g of the intended product.

PMR ($CDCl_3$) δ(ppm): 2.06(3H,s), 3.48-3.78(2H,m) 4.04(3H,s), 4.73-5.55(7H,m), 5.94(1H,q), 6.90(1H,s), 7.44(1H,d)

IR: 1786cm$^{-1}$ ($\nu$C=0)

Melting point: 110 - 118°C

Example 27

1-Methyl-2-propenyl 7-[2-(2-amino-4-thiazolyl)-2-methoxyiminoacetamido]-3-cephem-4-carboxylate

(A: 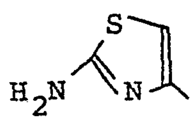, $R^1$: $CH_3$, $R^2$: H, $R^3$: 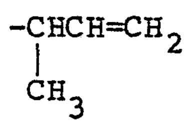)

Two grams of a molecular sieve (3A) was added to a solution of 0.77 g of 7-[2-(2-amino-4-thiazolyl)-2-methoxyiminoacetamido]-3-cephem-4-carboxylic acid in 8 ml of dry pyridine, and stirred at room temperature for 30 minutes. To the solution were added 1.5 ml of dry tetrahydrofuran and 0.16 g of 3-buten-2-ol and the resulting mixture was cooled with ice water. After adding 0.63 g of N,N'-dicyclohexylcarbodiimido to the mixture, the whole mixture was stirred first at an ice-cooled temperature for 4 hours and then at room temperature for 14 hours. The reaction mixture was further mixed with 40 ml of ethyl acetate and 40 ml of water. After adjusting

its pH to 4 with dilute hydrochloric acid, it was filtered. After removing the aqueous layer from the filtrate, the organic layer was washed with two 20 ml portions of 10% aqueous solution of dipotassium phosphate and then with 40 ml of saturated aqueous solution of sodium chloride. After drying it under a reduced pressure, the solvent was distilled off therefrom. Thus, 0.35 g of the intendet product was obtained.

PMR ($CDCl_3$) $\delta$ (ppm): 1.38 (3H, d), 3.45-3.56 (2H, m), 4.01 (3H, s), 5.00 (5H, m), 6.05 (1H, q), 6.49-6.59 (1H, m), 6.72 (1H, s), 7.78 (1H, d)

IR: 1790 $cm^{-1}$ ($\nu$C=O)

Melting point: 168 - 176°C

Example 28

2-Chloro-2-propenyl 7-[2-(2-amino-4-thiazolyl)-2-methoxyiminoacetamido]-3-cephem-4-carboxylate

(A: 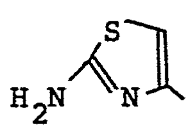 , $R^1$: $CH_3$ , $R^2$: H , $R^3$: 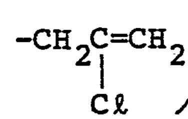 )

The intended product was produced by repeating the reaction and treatment of Example 3, except that the 3-chloro-2-methylpropene was replaced with 2,3-dichloro-1-propene.

PMR ($CDCl_3$) $\delta$ (ppm): 3.54 (2H, broad s), 4.03 (3H, s), 4.79 (2H, m), 5.05 (1H, d), 5.42 (1H, s), 5.53 (1H, s),

6.06(1H,q), 6.60(1H,m), 6.74(1H,s), 7.80(1H,d)

IR: $1785cm^{-1}$ ($\nu$C=O)

Melting poitn: 130 - 135°C

Example 29

4-Chloro-2-butenyl 7-[2-(2-amino-4-thiazolyl)-2-methoxyiminoacetamido]-3-cephem-4-carboxylate

(A: 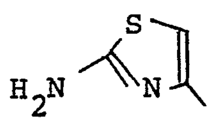 , $R^1$: $CH_3$ , $R^2$: H , $R^3$: $-CH_2CH{=}CHCH_2Cl$)

In 40 ml of acetone, 2.4 g of sodium iodide and 10 g of 1,4-dichloro-2-butene were heated under reflux for 1 hour. After filtration, the solvent was distilled off under reduced pressure. The residue was reacted in the same manner as in Example 3 to form the intended product.

PMR ($d_6$-DMSO) $\delta$(ppm): 3.57(2H, broad s), 4.38(2H,m), 4.70(2H,m), 5.08(1H,d), 5.5-6.1(3H,m), 6.5(1H,m), 6.68(1H,s), 7.15(2H, broad s), 9.55(1H,d)

IR: $1783cm^{-1}$ ($\nu$C=O)

Melting point: 180 - 185°C

Example 30

Allyl 7-[2-(2-amino-4-thiazolyl)-2-methoxyimino-acetamido]-3-acetoxymethyl-3-cephem-4-carboxylate

(A: 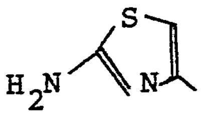 , $R^1$: $CH_3$ , $R^2$: $-CH_2OCOCH_3$ , $R^3$: $-CH_2CH{=}CH_2$ )

The intended product was produced in the same manner as in Example 1.

PMR($d_6$-DMSO) $\delta$(ppm): 2.02(3H,s), 3.59(2H, broad s), 3.82(3H,s), 4.6-5.4(7H,m), 5.7-6.1(2H,m), 6.71(1H,s), 7.18(2H,broad s), 9.55(1H,d)

IR: 1786 $cm^{-1}$ ($\nu_{C=O}$)

Melting point: 130 - 137°C

Example 31

2-Methyl-3-phenyl-2-propenyl 7-[2-(2-amino-4-thiazolyl)-2-methoxyiminoacetamido]-3-methyl-3-cephem-4-carboxylate

(A: 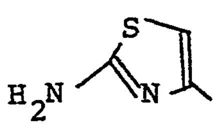 , $R^1$: $CH_3$ , $R^2$: $CH_3$ , $R^3$: 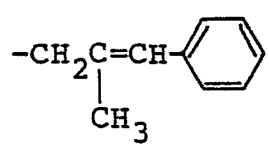 )

The intended product was produced by repeating the procedure of Example 16, except that the sodium 7-[2-(2-amino-4-thiazolyl)-2-methoxyiminoacetamido]-3-cephem-4-carboxylate was replaced with sodium 7-[2-(2-amino-4-thiazolyl)-2-methoxyiminoacetamido]-3-methyl-3-cephem-4-carboxylate.

PMR ($d_6$-DMSO) δ (ppm): 2.9-3.0 (6H,m), 3.48 (2H,m), 3.81 (3H,s), 4.73 (2H,m), 5.10 (1H,d), 5.71 (1H,q), 6.55 (1H,s), 6.69 (1H,s), 7.1-7.6 (5H,m), 9.52 (1H,d)

IR: 1780cm$^{-1}$ (νC=O)

Melting point: 90 - 100°C

Example 32

2-Methyl-3-(p-fluorophenyl)-2-propenyl 7-[2-(2-amino-4-thiazolyl)-2-methoxyiminoacetamido]-3-cephem-4-carboxylate

A: 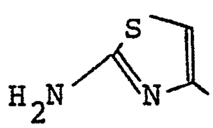 , $R^1$: $CH_3$ , $R^2$: H,

$R^3$: 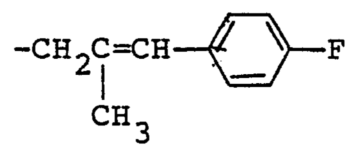

The intended product was produced by repeating the procedure of Example 16, except that the 1-chloro-2-methyl-3-phenyl-2-propene was replaced with 1-chloro-2-methyl-3-(p-fluorophenyl)-2-propene.

PMR ($CDCl_3$) δ (ppm): 1.9-2.0 (3H,m), 3.5-3.6 (2H,m), 4.03 (3H,s), 4.75-4.85 (2H,m), 5.05 (1H,d), 6.06 (1H,q), 6.5-6.6 (2H,m), 6.76 (1H,s), 6.9-7.3 (4H,m), 7.72 (1H,d)

IR: 1785cm$^{-1}$ (νC=O)

Melting point: 104 - 118°C

Example 33

2-Methyl-3-(o-tolyl)-2-propenyl 7-[2-(2-amino-4-thiazolyl)-2-methoxyiminoacetamido]-3-cephem-4-carboxylate

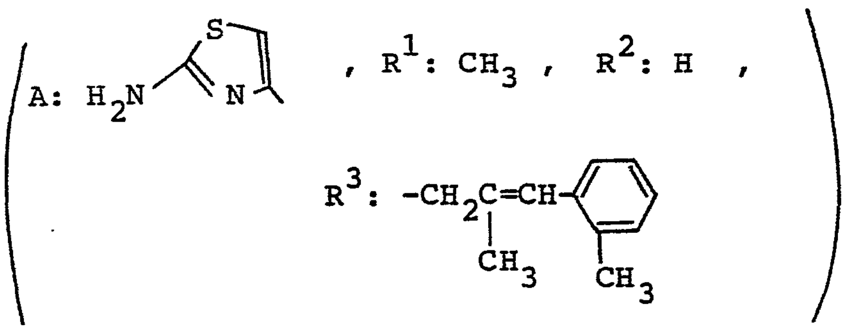

The intended product was produced by repeating the procedure of Example 16, except that the 1-chloro-2-methyl-3-phenyl-2-propene was replaced with 1-chloro-2-methyl-3-(o-tolyl)-2-propene.

PMR ($CDCl_3$) δ (ppm): 1.9-2.0 (3H, m), 2.33 (3H,s), 3.5-3.6 (2H,m), 4.04 (3H,s), 4.8-4.9 (2H,m), 5.05 (1H,d), 6.04 (1H,q), 6.5-6.6 (2H,m), 6.80 (1H,s), 7.0-7.1 (4H,m), 7.52 (1H,d)

IR: 1785cm$^{-1}$ ($\nu$C=0)

Melting point: 78 - 84°C

Example 34

2-Methyl-3-(p-anisyl)-2-propenyl 7-[2-(2-amino-4-thiazolyl)-2-methoxyiminoacetamido]-3-cephem-4-carboxylate

(A: 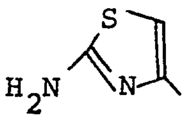 , $R^1$: $CH_3$ , $R^2$: H ,

$R^3$: 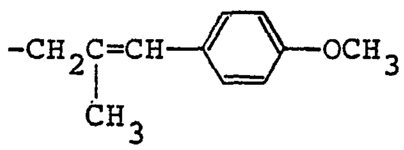 )

The intended product was produced by repeating the procedure of Example 16, except that the 1-chloro-2-methyl-3-phenyl-2-propene was replaced with 1-chloro-2-methyl-3-(p-anisyl)-2-propene.

PMR($CDC\ell_3$) δ(ppm): 1.9(3H,m), 3.5-3.6(2H,m), 3.80(3H,s), 4.05(3H,s), 4.8-4.9(2H,m), 5.05(1H,d), 6.05 (1H,q), 6.5-6.6(2H,m), 6.8-7.2(5H,m), 7.50(1H,d)

IR: 1790$cm^{-1}$ ($\nu$C=0)

Melting poitn: 110 - 117°C

Example 35

2-Methyl-3-(m-tolyl)-2-propenyl 7-[2-(2-amino-4-thiazolyl)-2-methoxyiminoacetamido]-3-cephem-4-carboxylate

(A: 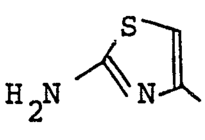 , $R^1$: $CH_3$ , $R^2$: H ,

$R^3$: 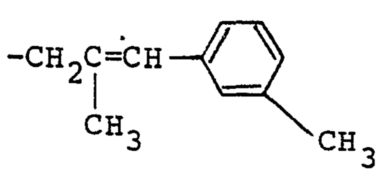 )

The intended product was produced by repeating the procedure of Example 16, except that the 1-chloro-2-methyl-3-phenyl-2-propene was replaced with 1-chloro-2-methyl-3-(m-tolyl)-2-propene.

PMR($CDCl_3$) δ(ppm): 1.8-2.0(3H,m), 2.13(3H,s), 3.5-3.6(2H,m), 4.06(3H,s), 4.7-4.8(2H,m), 5.0-5.1(1H,m), 6.05(1H,q), 6.4-6.6(2H,m), 6.81(1H,s), 7.0-7.1(4H,m), 7.54(1H,d)

IR: $1785cm^{-1}$ ($\nu$C=O)

Melting pointL 109 - 118°C

Example 36

2-Methyl-3-(m-anisyl)-2-propenyl 7-[2-(2-amino-4-thiazolyl)-2-methoxyiminoacetamido]-3-cephem-4-carboxylate

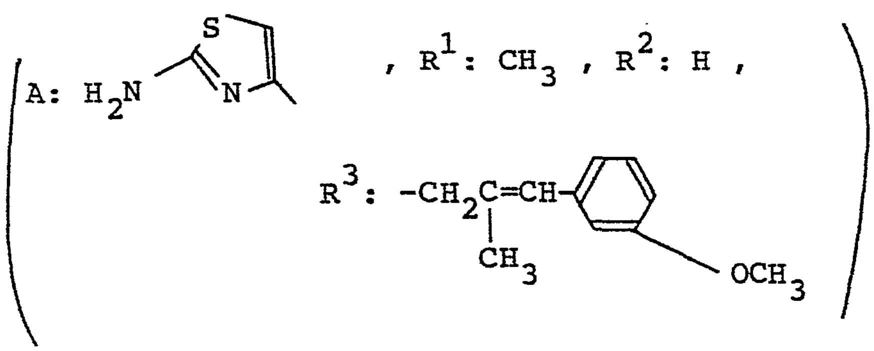

The intended product was produced by repeating the procedure of Example 16, except that the 1-chloro-2-methyl-3-phenyl-2-propene was replaced with 1-chloro-2-methyl-3-(m-anisyl)-2-propene.

PMR($CDCl_3$) δ(ppm): 1.9-2.0(3H,m), 3.4-3.6 (2H,m), 3.77(3H,d), 4.03(3H,s), 4.7-4.9(2H,m), 5.04(1H,d),

6.05(1H,q), 6.5-6.6(2H,m), 6.6-6.9(4H,m), 7.1-7.3(1H,m), 7.74(1H,d)

IR: 1785cm$^{-1}$ ($\nu$C=0)

Melting point: 90 - 104°C

Example 37

2-Methyl-3-(p-tolyl)-2-propenyl 7-[2-(2-amino-4-thiazolyl)-2-methoxyiminoacetamido]-3-cephem-4-carboxylate

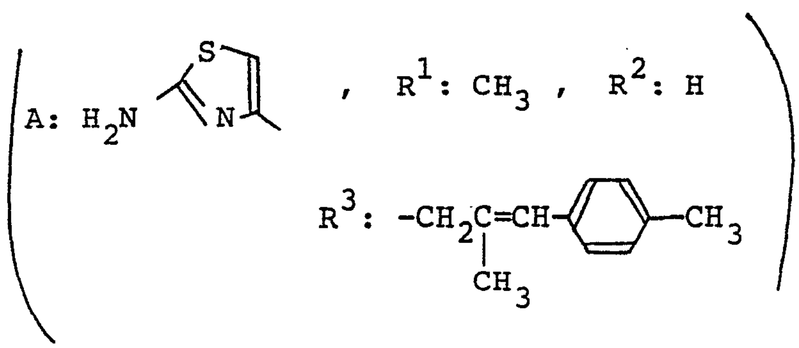

The intended product was produced by repeating the procedure of Example 16, except that the 1-chlolo-2-methyl-3-phenyl-2-propene was replaced with 1-chloro-2-methyl-3-(p-tolyl)-2-propene.

PMR($CDCl_3$) $\delta$(ppm): 1.9-1.95(3H,m), 2.33(3H,s), 3.4-3.6(2H,m), 4.04(3H,s), 4.8-4.9(2H,m), 5.05(1H,d), 6.06(1H,q), 6.5-6.6(2H,m), 6.77(1H,s), 7.0-7.2(4H,m), 7.65(1H,d)

IR: 1795 cm$^{-1}$ ($\nu_{C=O}$)

Melting point: 106 - 109°C

Example 38

2-Methyl-3-(o-anisyl)-2-propenyl 7-[2-(2-amino-4-thiazolyl)-2-methoxyiminoacetamido]-3-cephem-4-carboxylate

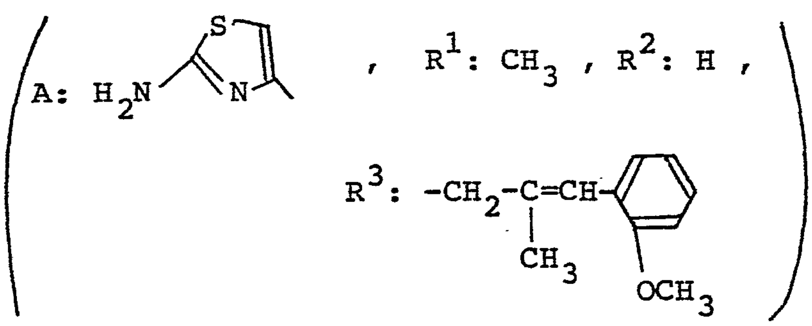

The intended product was produced by repeating the procedure of Example 16, except that the 1-chloro-2-methyl-3-phenyl-2-propene was replaced with 1-chloro-2-methyl-3-(o-anisyl)-2-propene.

PMR($CDCl_3$) δ(ppm): 1.9-2.0(3H,m), 3.5-3.6 (2H,m), 3.81(1H,s), 4.04(3H,s), 4.84(2H,s), 5.06(1H,d), 6.06(1H,q), 6.5-6.7(2H,m), 6.79(1H,s), 6.8-7.3(4H,m), 7.65(1H,d)

IR: $1790cm^{-1}$ ($\nu$C=O)

Melting point: 99 - 105°C

Example 39

2-Ethyl-2-hexenyl 7-[2-(2-amino-4-thiazolyl)-2-methoxyiminoacetamido]-3-cephem-4-carboxylate

(A: 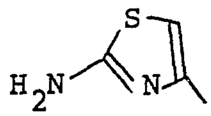 , $R^1$: $CH_3$ , $R^2$: H ,

$R^3$: 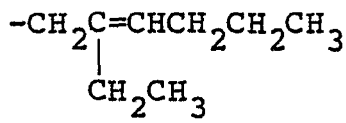 )

In 10 ml of acetone, 0.95 g of 1-chloro-2-ethyl-2-hexene and 0.98 g of sodium iodide were heated under reflux for 30 minutes. The resulting crystal was filtered off, and the acetone was carefully distilled off from the filtrate. Subsequently, 20 ml of dimethylformamide and 2.03 g of sodium 7-[2-(2-amino-4-thiazolyl)-2-methoxyiminoacetamido]-3-cephem-4-carboxylate were added to the residue and reacted for 18 hours. The reaction mixture was poured into a mixture consisting of 200 ml of ethyl acetate and 50 ml of aqueous hydrochloric acid having a pH value of 3.0 and stirred. After separation into layers, the ethyl acetate layer was washed successively with 50 ml of aqueous hydrochloric acid having a pH value of 3.0, two 50 ml portions of phosphate buffer solution having a pH value of 7.0 and 50 ml of saturated aqueous solution of sodium chloride.

Subsequently, it was dried over anhydrous magnesium sulfate and concentrated under reduced pressure. The residue was crystallized by dropping it into 400 ml of petroleum ether. The crystalline product was collected by filtration, washed with petroleum ether and then dried

under reduced pressure. Thus, 1.50 g of the intended product was obtained.

PMR($d_6$-DMSO) δ(ppm): 0.8-1.1(6H,m), 1.1-1.6 (2H,m), 1.8-2.4(4H,m), 3.6(2H,m), 3.82(3H,s), 4.7(2H,m), 5.10(1H,d), 5.42(1H,q), 5.80(1H,q), 6.5(1H,m), 6.68(1H,s), 7.2(2H,s), 9.54(1H,d)

IR: 1785$cm^{-1}$ ($\nu$C=O)

Melting point: 71 - 86°C

Example 40

2-Methyl-2-pentenyl 7-[2-(2-amino-4-thiazolyl)-2-methoxyiminoacetamido]-3-cephem-4-carbosylate

(A: 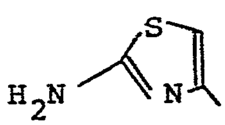 , $R^1$: $CH_3$ , $R^2$: H ,

$R^3$: 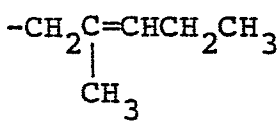 )

The intended-product was produced by repeating the procedure of Example 39, except that the 1-chloro-2-ethyl-2-hexene was replaced with 1-chloro-2-methyl-2-pentane.

PMR($d_6$-DMSO) δ(ppm): 0.91(3H,t), 1.7(3H,m), 1.9-2.3(2H,m), 3.6(2H,m), 3.81(3H,s), 4.6(2H,m), 5.05 (1H,d), 5.43(1H,q), 5.82(1H,q), 6.51(1H,m), 6.69(1H,s), 7.2(2H,s), 9.55(1H,d)

IR: 1780$cm^{-1}$ ($\nu$C=O)

Melting point: 93 - 104°C

Example 41

2,3-Dimethyl-2-butenyl 7-[2-(2-amino-4-thiazolyl)-2-methoxyiminoacetamido]-3-cephem-4-carboxylate

( A: 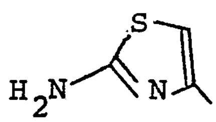 , $R^1$: $CH_3$ , $R^2$: H ,

$R^3$: 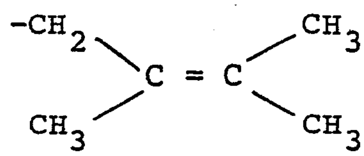 )

The intended product was produced by repeating the procedure of Example 39, except that the 1-chloro-2-ethyl-2-hexene was replaced with 1-bromo-2,3-dimethyl-2-butene.

PMR($d_6$-DMSO) δ(ppm): 1.7(9H,m), 3.6(2H,m), 3.82(3H,s), 4.69(2H,s), 5.10(1H,d), 5.82(1H,q), 6.48(1H,t), 6.69(1H,s), 7.17(2H,s), 9.55(1H,d)

IR: 1785cm$^{-1}$ ($\nu$C=0)

Melting point: 104 - 117°C

Example 42

2-Methyl-2-butenyl 7-[2-(2-amino-4-thiazolyl)-2-methoxyiminoacetamido]-3-cephem-4-carboxylate

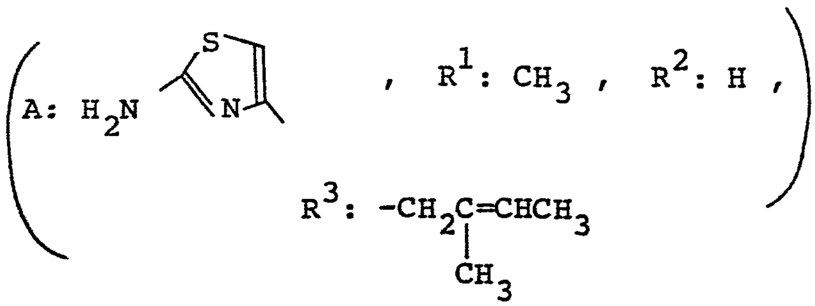

The intended product was produced by repeating the procedure of Example 39, except that the 1-chloro-2-ethyl-2-hexene was replaced with 1-chloro-2-methyl-2-butene.

PMR ($d_6$-DMSO) δ (ppm): 1.6-1.7 (6H, m), 3.6 (2H, m), 3.83 (3H, s), 4.6 (2H, m), 5.10 (1H, d), 5.3-5.6 (1H, m), 5.82 (1H, q), 6.51 (1H, t), 6.69 (1H, s), 7.2 (2H, s), 9.55 (1H, d)

IR: 1785 $cm^{-1}$ ($\nu$C=O)

Melting point: 93 - 107°C

Example 43

2-Ethyl-3-phenyl-2-propenyl 7-[2-(2-amino-4-thiazolyl)-2-methoxyiminoacetamido]-3-cephem-4-carboxylate

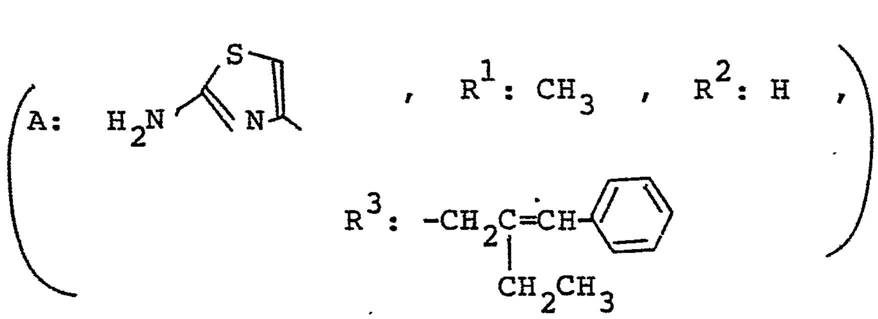

The intended product was produced by repeating the procedure of Example 16, except that the 1-chloro-2-methyl-3-phenyl-2-propene was replaced with 1-chloro-2-ethyl-3-phenyl-2-propene.

PMR ($d_6$-DMSO) δ (ppm): 1.10 (3H, t), 2.30 (2H, q), 3.6 (2H, m), 3.82 (3H, s), 4,8 (2H, s), 5.10 (1H, d), 5.92 (1H, q), 6.4-6.6 (2H, m), 6.68 (1H, s), 7.1-7.3 (7H, m), 9.55 (1H, d)

IR: 1780cm$^{-1}$

Melting point: 84 - 94°C

Example 44

2-Methyl-2-propenyl 7-[2-methoxyimino-2-(4-thiazalyl)-acetemido]-3-(1,3,4-thiadiazol-2-yl)-thiomethyl-3-cephem-4-carboxylate

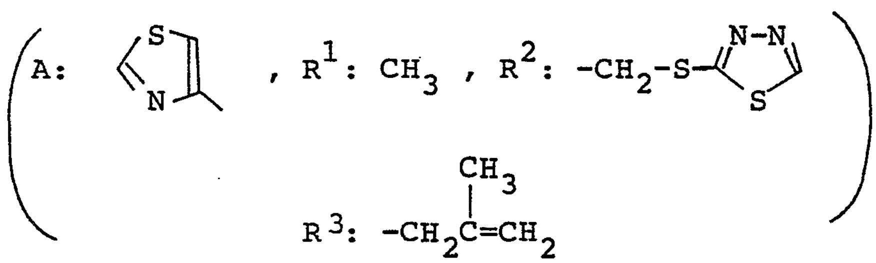

The intended product was produced by repeating the procedure of Example 3, except that the sodium 7-[2-(2-amino-4-thiazolyl)-2-methoxyiminoacetamido]-3-cephem-4-carboxylate was replaced with sodium 7-[2-methoxyimino-2-(4-thiazolyl)-acetamido]-3-(1,3,4-thiadiazol-2-yl)-thiomethyl-3-cephem-4-carboxylate.

PMR ($d_6$-DMSO) δ (ppm): 1.75 (3H, s), 3.74 (2H, q), 3.91 (3H, s), 4.2-4.7 (2H, q), 4.65 (2H, s), 4.9-5.1 (2H, m),

5.20(1H,d), 5.89(1H,q), 7.91(1H,d), 9.14(1H,d), 9.52(1H,s), 9.68(1H,d)

Example 45

2-Methyl-2-propenyl 7-[2-methoxyimino-2-(4-thiazolyl)-acetamido]-3-acetoxymethyl-3-cephem-4-carboxylate

( A: 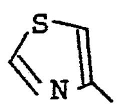 , $R^1$: $CH_3$ , $R^2$: 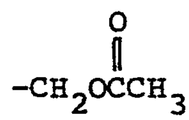

$R^3$: 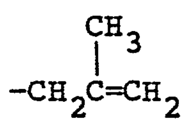 )

The intended product was produced by repeating the procedure of Example 3, except that the sodium 7-[2-(2-amino-4-thiazolyl)-2-methoxyiminoacetamido]-3-cephem-4-carboxylate was replaced with sodium 7-[2-methoxyimino-2-(4-thiazolyl)-acetamido]-3-acetoxymethyl-3-cephem-4-carboxylate.

PMR($d_6$-DMSO) δ(ppm); 1.71(3H,s), 2.00(3H,s), 3.60(2H,ABq), 3.89(3H,s), 4.63(2H,s), 4.66 and 4.94 (2H,ABq), 4.9-5.1(2H,m), 5.20(1H,d), 5.87(1H,q), 7.91 (1H,d), 9.13(1H,d), 9.66(1H,d)

CLAIMS FOR ALL DESIGNATED STATES OTHER THAN AUSTRIA

1. A compound represented by the general formula I

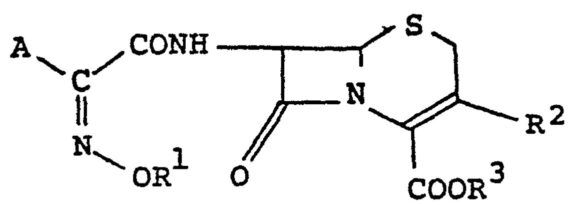

I

wherein A represents an unsubstituted thiazolyl residue or a substituted thiazolyl residue having at least one substituent which is halogen or a lower alkyl, amino or acylamido residue; $R^1$ represents hydrogen or a lower alkyl, carboxymethyl or lower alkoxycarbonylmethyl residue; $R^2$ represents hydrogen or a methyl, hydroxymethyl, acyloxymethyl or lower alkylthiomethyl residue, or represents a thiadiazolylthiomethyl residue optionally substituted by at least one lower alkyl residue, or represents a tetrazolylthiomethyl residue optionally substituted by at least one lower alkyl, lower alkylaminoalkyl or carboxymethyl residue; and $R^3$ represents an unsubstituted allyl residue or a substituted allyl residue having at least one substituent which is halogen or a lower alkyl, lower cycloalkyl, lower haloalkyl, acyloxy-(lower alkyl), lower alkoxycarbonyl, phenyl or substituted phenyl residue; or a salt thereof.

2. A compound according to claim 1 represented by the general formula:

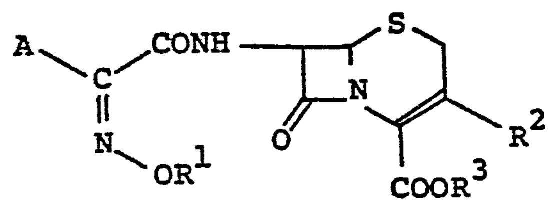

wherein A is an unsubstituted or amino-substituted thiazolyl residue; $R^1$ is hydrogen or a lower alkyl or carboxymethyl residue; $R^2$ is a thiadiazolylthiomethyl residue optionally substituted by a methyl or other lower alkyl residue, or is a tetrazolylthiomethyl residue optionally substituted by a lower alkyl residue; and $R^3$ is allyl residue having a substituent which is halogen or a lower alkyl, phenyl or substituted phenyl residue; or a salt thereof.

3. A compound according to claim 1 represented by the general formula:

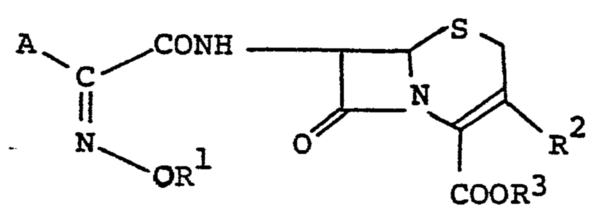

wherein A is a residue represented by formula 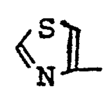

or formula 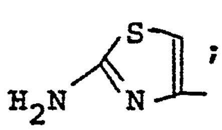 ; $R^1$ is hydrogen or a methyl or carboxymethyl residue; $R^2$ is hydrogen or a methyl residue or a residue represented by formula

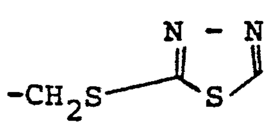 , 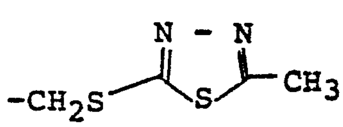 or 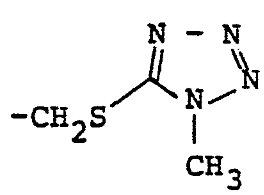 ; and

$R^3$ is allyl residue having a substituent which is chlorine or a methyl, ethyl, propyl, phenyl or substituted phenyl residue optionally substituted by a lower alkyl, lower alkoxy residue or halogen; or a salt thereof.

4. A compound or salt according to claim 1 hereinbefore specifically mentioned.

5. A process for producing a compound as defined in any one of the preceding claims comprising reacting a compound represented by the general formula:

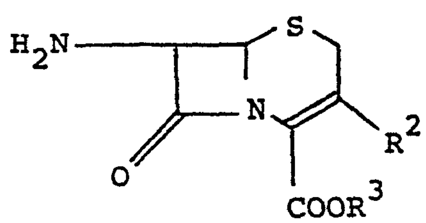

wherein $R^2$ and $R^3$ are as defined in any one of the preceding claims, or its reactive derivative at the amino derivative or salt thereof with a compound represented by the general formula:

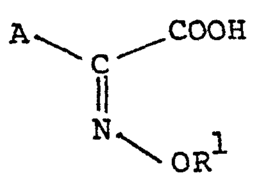

wherein A and $R^1$ are as defined in any one of the preceding claims, or its reactive derivative at the carboxyl residue or salt thereof.

6. A process for producing a compound or salt as defined in any one of claims 1 to 4 comprising reacting a compound represented by the general formula:

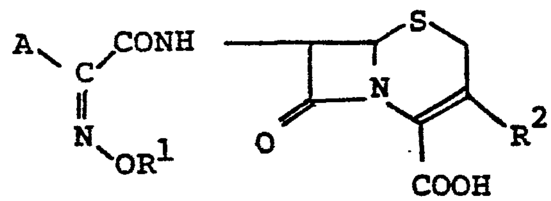

wherein A, $R^1$ and $R^2$ are as defined in any one of claims 1 to 4, or its reactive derivative at the carboxyl residue or salt thereof with an esterifying agent represented by the general formula:

$$R^3 - B$$

wherein B represents a hydroxyl residue or its reactive derivative residue, and $R^3$ is as defined in any one of claims 1 to 4.

7. A process for producing a compound or salt as defined in any one of claims 1 to 4 comprising reacting an etherifying agent upon a compound represented by the general formula:

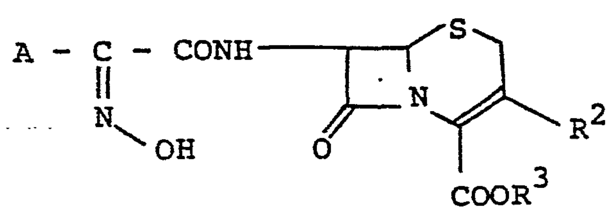

wherein A, $R^2$ and $R^3$ are as defined in any one of claims 1 to 4.

8. A process for producing a compound or salt as defined in any one of claims 1 to 4 comprising reacting a compound represented by the general formula:

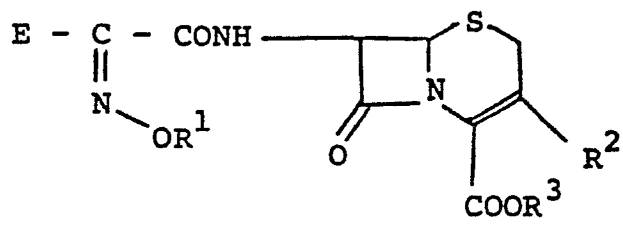

wherein E represents a haloacetyl residue, and $R^1$, $R^2$ and $R^3$ are as defined in any one of claims 1 to 4, or a salt thereof with a compound represented by the general formula:

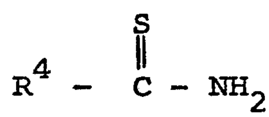

wherein $R^4$ represents hydrogen or halogen, or a lower alkyl, amino or acylamido residue; or salt thereof.

9. A pharmaceutical composition comprising an effective amount of the compound or salt according to any one of claims 1 to 4 and a pharmacologically acceptable carrier or diluent.

10. A compound or salt according to any one of claims 1 to 4 or a composition according to claim 8 for use in a method for treatment of the human or animal body by surgery or therapy or a method of diagnosis practised on the human or animal body.

CLAIMS FOR AUSTRIA

1. A process for producing a compound represented by the general formula I:

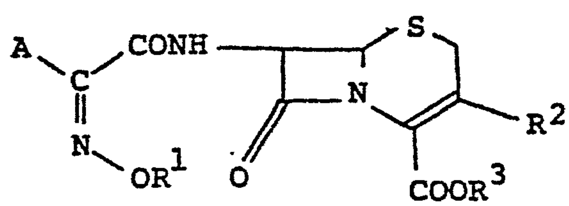

I

wherein A represents an unsubstituted thiazolyl residue or a substituted thiazolyl residue having at least one substituent which is halogen or a lower alkyl, amino or acylamido residue; $R^1$ represents hydrogen or a lower alkyl, carboxymethyl or lower alkoxycarbonylmethyl residue; $R^2$ represents hydrogen or a methyl, hydroxymethyl, acyloxymethyl or lower alkylthiomethyl residue, or represents a thiadiazolylthiomethyl residue optionally substituted by at least one lower alkyl residue, or represents a tetrazolylthiomethyl residue optionally substituted by at least one lower alkyl, lower alkylaminoalkyl or carboxymethyl residue; and $R^3$ represents an unsubstituted allyl residue or a substituted allyl residue having at least one substituent which is halogen or a lower alkyl, lower cycloalkyl, lower haloalkyl, acyloxy-(lower alkyl), lower alkoxycarbonyl, phenyl or substituted phenyl residue; or a salt thereof, comprising:

(a) reacting a compound represented by the general formula:

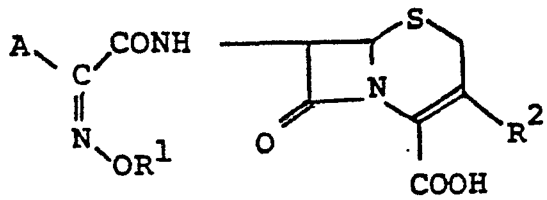

wherein A, $R^1$ and $R^2$ are as defined above, or its reactive derivative at the carboxyl residue or salt thereof with an esterifying agent represented by the general formula:

$$R^3 - B$$

wherein B represents a hydroxyl residue or its reactive derivative residue, and $R^3$ is as defined above;

or (b) reacting an etherifying agent upon a compound represented by the general formula:

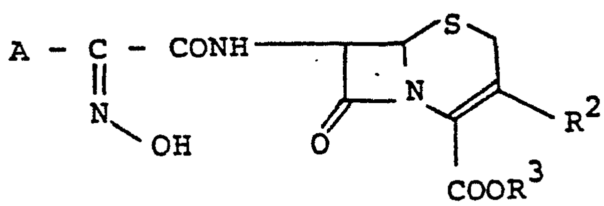

wherein A, $R^2$ and $R^3$ are as defined above;

or (c) reacting a compound represented by the general formula:

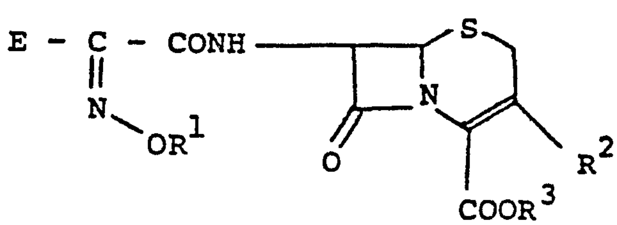

wherein E represents a haloacetyl residue, and $R^1$, $R^2$ and $R^3$ are as defined above, or a salt thereof with a compound represented by the general formula:

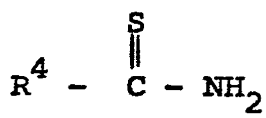

wherein $R^4$ represents hydrogen or halogen, or a lower alkyl, amino or acylamido residue.

2. A process according to claim 1 wherein a compound is prepared represented by the general formula:

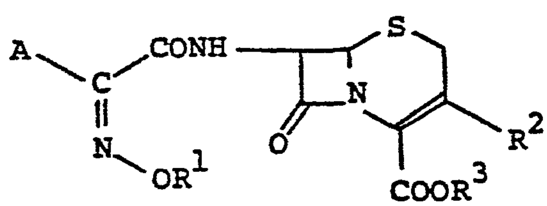

wherein A is an unsubstituted or amino-substituted thiazolyl residue; $R^1$ is hydrogen or a lower alkyl or carboxymethyl residue; $R^2$ is a thiadiazolylthiomethyl residue optionally substituted by a methyl or other lower alkyl residue, or is a tetrazolylthiomethyl residue optionally substituted by a lower alkyl residue; and $R^3$ is allyl residue having a substituent which is halogen or a lower alkyl, phenyl or substituted phenyl residue; or a salt thereof.

3. A process according to claim 1 wherein a compound is prepared represented by the general formula:

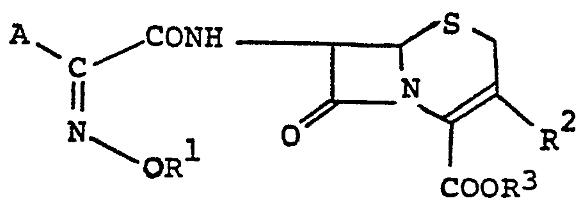

wherein A is a residue represented by formula 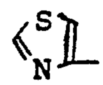

or formula 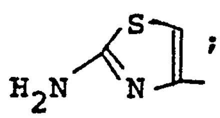 ; $R^1$ is hydrogen or a methyl or carboxymethyl residue; $R^2$ is hydrogen or a methyl residue or a residue represented by formula

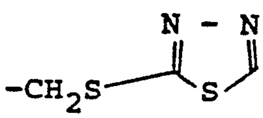 , 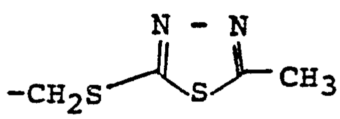 or 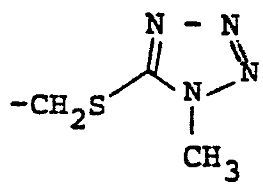 ; and

$R^3$ is allyl residue having a substituent which is chlorine or a methyl, ethyl, propyl, phenyl or substituted phenyl residue optionally substituted by a lower alkyl, lower alkoxy residue or halogen; or a salt thereof.